# EUROPEAN PATENT SPECIFICATION

(11) **EP 1 448 984 B1**
(45) Date of publication and mention of the grant of the patent: **14.03.2012**
(21) Application number: 02784565.0
(22) Date of filing: 23.11.2002
(51) Int. Cl.: G01N 31/00, G01N 33/48, A61K 38/02

(54) **SURFACE SIMULATION SYNTHETIC PEPTIDES USEFUL IN THE TREATMENT OF HYPER-VARIABLE VIRAL PATHOGENS**
ZUR BEHANDLUNG HYPERVARIABLER VIRALER KRANKHEITSERREGER GEEIGNETE SYNTHETISCHE OBERFLÄCHENSIMULATIONSPEPTIDE
PEPTIDES SYNTHETIQUES DE SIMULATION DE SURFACE UTILES DANS LE TRAITEMENT DE PATHOGENES VIRAUX HYPER VARIABLES

(30) Priority: 01.12.2001 US 12806
(43) Date of publication of application: 25.08.2004
(73) Proprietor: Crevecoeur, Harry, Valley Stream, NY 11581 (US)
(72) Inventor: Crevecoeur, Harry, Valley Stream, NY 11581 (US)
(74) Representative: Barz, Peter
(86) International application number: PCT/US2002/037664
(87) International publication number: WO 2003/048186

(56) References cited:
- WO-A-99/24553
- WO-A1-01/09191
- KWONG P D ET AL: "STRUCTURE OF AN HIV GP 120 ENVELOPE GLYCOPROTEIN IN COMPLEX WITH THE CD4 RECEPTOR AND A NEUTRALIZING HUMAN ANTIBODY" NATURE, NATURE PUBLISHING GROUP, LONDON, GB, vol. 393, 1998, pages 648-659, XP000882889 ISSN: 0028-0836
- RIZZUTO C D ET AL: "CONSERVED HIV GP 120 GLYCOPROTEIN STRUCTURE INVOLVED IN CHEMOKINE RECEPTOR BINDING" SCIENCE, AMERICAN ASSOCIATION FOR THE ADVANCEMENT OF SCIENCE,, US, vol. 280, 19 June 1998 (1998-06-19), pages 1949-1953, XP000882888 ISSN: 0036-8075
- TAYLOR K M ET AL: "Influence of three-dimensional structure on the immunogenicity of a peptide expressed on the surface of a plant virus." JOURNAL OF MOLECULAR RECOGNITION : JMR. 2000 MAR-APR, vol. 13, no. 2, March 2000 (2000-03), pages 71-82, XP002391692 ISSN: 0952-3499
- THALI ET AL: "Discontinuous, conserved neutralization epitopes overlapping the CD4-binding region of human immunodeficiency virus type 1 gp120 envelope glycoprotein" JOURNAL OF VIROLOGY, NEW YORK, US, US, vol. 66, no. 9, September 1992 (1992-09), pages 5635-5641, XP002119923 ISSN: 0022-538X
- VILLEN JUDIT ET AL: "Synthetic peptides as functional mimics of a viral discontinuous antigenic site" BIOLOGICALS, vol. 29, no. 3-4, September 2001 (2001-09), pages 265-269, XP002391920 ISSN: 1045-1056
- TWINING S S ET AL: "ANTIBODY COMBINING SITES CAN BE MIMICKED SYNTHETICALLY SURFACE SIMULATION SYNTHESIS OF THE IMMUNO GLOBULIN NEW COMBINING SITE TO THE GAMMA HYDROXYL DERIVATIVE OF VITAMIN K-1" JOURNAL OF BIOLOGICAL CHEMISTRY, vol. 253, no. 15, 1978, pages 5259-5262, XP002391921 ISSN: 0021-9258
- KELKER H C ET AL: "Immunogenic and antigenic properties of an HIV-1 gp120-derived multiple chain peptide." JOURNAL OF IMMUNOLOGY (BALTIMORE, MD. : 1950) 15 APR 1994, vol. 152, no. 8, 15 April 1994 (1994-04-15), pages 4139-4148, XP002391693 ISSN: 0022-1767
- FERRER M ET AL: "Structural and functional characterization of an epitope in the conserved C-terminal region of HIV-1 gp120" JOURNAL OF PEPTIDE RESEARCH, BLACKWELL PUBLISHING LTD., OXFORD, GB, vol. 54, no. 1, July 1999 (1999-07), pages 32-42, XP002292693 ISSN: 1397-002X
- ZENG J ET AL: "Design of inhibitors of Ras--Raf interaction using a computational combinatorial algorithm." PROTEIN ENGINEERING. JAN 2001, vol. 14, no. 1, January 2001 (2001-01), pages 39-45, XP002391694 ISSN: 0269-2139
- FAIRLIE D. ET AL.: 'Towards protein surface mimetics' CURRENT MEDICINAL CHEMISTRY vol. 5, no. 1, 1998, pages 29 - 62, XP000983590
- BIENSTOCK R. ET AL.: 'Molecular modeling of protein structures' SCIENCE & MEDICINE vol. 4, no. 1, 1997, pages 54 - 63, XP002972041
- VITA ET AL.: 'Novel miniprotein engineered by the transfer of active sites to small natural scaffolds' BIOPOLYMERS vol. 47, no. 1, 1998, pages 93 - 100, XP002924847

### SEQUENCE LISTING

<110> CREVICOUER, HARRY
<120> SURFACE SIMULATION SYNTHETIC PEPTIDES USEFUL IN THE TREATMENT OF HYPER-VARIABLE VIRAL PATHOGENS
<130> Crevl PCT
<190>
   <141>
<160> 45
<170> PatentIn Ver. 2.1
<210> 1
   <211> 13
   <212> PRT
   <213> Artificial Sequence
<220>
   <223> Description of Artificial Sequence: Synthetic peptide
<400> 1
   Asp Trp Glu Thr Cys Asn Asn Cys Arg Lys Glu Asp Trp
    1               5                   10
<210> 2
   <211> 13
   <212> PRT
   <213> Artificial Sequence
<220>
   <223> Description of Artificial Sequence: Synthetic peptide
<400> 2
   Asn Cys Arg Lys Glu Asp Trp Asp Glu Lys Arg Cys Asn
   1 5 10
<210> 3
   <211> 11
   <212> PRT
   <213> Artificial Sequence
<220>
   <223> Description of Artificial Sequence: Synthetic peptide
<400> 3
   Asp Trp Glu Thr Cys Asn Cys Thr Glu Trp Asp
   1 5 10
<210> 4
   <211> 16
   <212> PRT
   <213> Artificial Sequence
<220>
   <223> Description of Artificial Sequence: Synthetic peptide
<400> 4
   Trp Asp Glu Thr Cys Asn Cys Thr Glu Asp Trp Asp Glu Thr Cys Asn
   1 5 10 15
<210> 5
   <211> 15
   <212> PRT
   <213> Artificial Sequence
<220>
   <223> Description of Artificial Sequence: Synthetic peptide
<400> 5
   Asn Cys Arg Thr Glu Lys Gln Trp Gln Lys Glu Thr Arg Cys Asn
   1 5 10 15
<210> 6
   <211> 8
   <212> PRT
   <213> Artificial Sequence
<220>
   <223> Description of Artificial Sequence: Synthetic peptide
<400> 6
   Gln Trp Lys Glu Arg Thr Cys Asn
   1 5
<210> 7
   <211> 10
   <212> PRT
   <213> Artificial Sequence
<220>
   <223> Description of Artificial Sequence: Synthetic peptide
<400> 7
   Gln Lys Trp Glu Thr Cys Cys Arg Lys Trp
   1 5 10
<210> 8
   <211> 11
   <212> PRT
   <213> Artificial Sequence
<220>
   <223> Description of Artificial Sequence: Synthetic peptide
<400> 8
   Gln Lys Trp Asp Glu Thr Cys Cys Arg Lys Trp
   1 5 10
<210> 9
   <211> 6
   <212> PRT
   <213> Artificial Sequence
<220>
   <223> Description of Artificial Sequence: Synthetic peptide
<400> 9
   Asp Trp Glu Thr Cys Asn
   1 5
<210> 10
   <211> 11
   <212> PRT
   <213> Artificial Sequence
<220>
   <223> Description of Artificial Sequence: Synthetic peptide
<400> 10
   Asp Trp Glu Thr Cys Cys Arg Lys Gln Trp Lys
   1 5 10
<210> 11
   <211> 9
   <212> PRT
   <213> Artificial Sequence
<220>
   <223> Description of Artificial Sequence: Synthetic peptide
<400> 11
   Gln Lys Arg Cys Cys Thr Glu Asp Trp
   1 5
<210> 12
   <211> 14
   <212> PRT
   <213> Artificial Sequence
<220>
   <223> Description of Artificial Sequence: Synthetic peptide
<400> 12
   Gln Lys Arg Cys Cys Asn Thr Glu Asp Trp Ile Ile Met Gln
   1 5 10
<210> 13
   <211> 8
   <212> PRT
   <213> Artificial Sequence
<220>
   <223> Description of Artificial Sequence: Synthetic peptide
<400> 13
   Gln Lys Arg Cys Cys Thr Glu Asp
   1 5
<210> 14
   <211> 13
   <212> PRT
   <213> Artificial Sequence
<220>
   <223> Description of Artificial Sequence: Synthetic peptide
<400> 14
   Asp Trp Glu Lys Arg Cys Cys Asn Thr Glu Asp Trp Lys
   1 5 10
<210> 15
   <211> 8
   <212> PRT
   <213> Artificial Sequence
<220>
   <223> Description of Artificial Sequence: Synthetic peptide
<400> 15
   Gln Lys Arg Cys Cys Thr Glu Asp
   1 5
<210> 16
   <211> 10
   <212> PRT
   <213> Artificial Sequence
<220>
   <223> Description of Artificial Sequence: Synthetic peptide
<400> 16
   Trp Gln Lys Arg Cys Cys Thr Glu Asp Trp
   1 5 10
<210> 17
   <211> 8
   <212> PRT
   <213> Artificial Sequence
<220>
   <223> Description of Artificial Sequence: Synthetic peptide
<400> 17
   Lys Trp Glu Thr Cys Cys Arg Lys
   1 5
<210> 18
   <211> 9
   <212> PRT
   <213> Artificial Sequence
<220>
   <223> Description of Artificial Sequence: Synthetic peptide
<400> 18
   Lys Trp Glu Thr Cys Cys Arg Lys Gln
   1 5
<210> 19
   <211> 8
   <212> PRT
   <213> Artificial Sequence
<220>
   <223> Description of Artificial Sequence: Synthetic peptide
<400> 19
   Thr Asn Cys Cys Arg Lys Glu Thr
   1 5
<210> 20
   <211> 12
   <212> PRT
   <213> Artificial Sequence
<220>
   <223> Description of Artificial Sequence: Synthetic peptide
<400> 20
   Asp Trp Glu Thr Asn Cys Cys Arg Lys Gln Trp Lys
   1 5 10
<210> 21
   <211> 14
   <212> PRT
   <213> Artificial Sequence
<220>
   <223> Description of Artificial Sequence: Synthetic peptide
<400> 21
   Ile Trp Asp Glu Thr Asn Cys Cys Arg Lys Gln Ile Trp Lys
   1 5 10
<210> 22
   <211> 15
   <212> PRT
   <213> Artificial Sequence
<220>
   <223> Description of Artificial Sequence: Synthetic peptide
<400> 22
   Ile Trp Asp Glu Thr Asn Cys Cys Arg Lys Gln Asn Ile Trp Lys
   1 5 10 15
<210> 23
   <211> 9
   <212> PRT
   <213> Artificial Sequence
<220>
   <223> Description of Artificial Sequence: Synthetic peptide
<400> 23
   Leu Pro Cys Arg Lys Gln Met Ile Trp
   1 5
<210> 24
   <211> 15
   <212> PRT
   <213> Artificial Sequence
<220>
   <223> Description of Artificial Sequence: Synthetic peptide
<400> 24
   Leu Pro Cys Arg Lys Gln Met Ile Trp Asp Trp Glu Thr Asn Cys
   1 5 10 15
<210> 25
   <211> 10
   <212> PRT
   <213> Artificial Sequence
<220>
   <223> Description of Artificial Sequence: Synthetic peptide
<400> 25
   Leu Pro Cys Arg Ile Lys Gln Met Ile Trp
   1 5 10
<210> 26
   <211> 16
   <212> PRT
   <213> Artificial Sequence
<220>
   <223> Description of Artificial Sequence: Synthetic peptide
<400> 26
   Leu Pro Cys Arg Ile Lys Gln Met Ile Trp Asp Trp Glu Thr Asn Cys
   1 5 10 15
<210> 27
   <211> 14
   <212> PRT
   <213> Artificial Sequence
<220>
   <223> Description of Artificial Sequence: Synthetic peptide
<400> 27
   Leu Pro Cys Arg Lys Gln Trp Asp Trp Glu Thr Cys Asn Cys
   1 5 10
<210> 28
   <211> 8
   <212> PRT
   <213> Artificial Sequence
<220>
   <223> Description of Artificial Sequence: Synthetic peptide
<400> 28
   Leu Pro Cys Cys Thr Glu Trp Asp
   1 5
<210> 29
   <211> 9
   <212> PRT
   <213> Artificial Sequence
<220>
   <223> Description of Artificial Sequence: Synthetic peptide
<400> 29
   Leu Pro Cys Cys Thr Glu Trp Lys Arg
   1 5
<210> 30
   <211> 8
   <212> PRT
   <213> Artificial Sequence
<220>
   <223> Description of Artificial Sequence: Synthetic peptide
<400> 30
   Leu Pro Cys Cys Cys Cys Arg Lys
   1 5
<210> 31
   <211> 14
   <212> PRT
   <213> Artificial Sequence
<220>
   <223> Description of Artificial Sequence: Synthetic peptide
<400> 31
   Cys Cys Cys Cys Arg Lys Trp Glu Thr Cys Cys Cys Cys Asn
   1 5 10
<210> 32
   <211> 16
   <212> PRT
   <213> Artificial Sequence
<220>
   <223> Description of Artificial Sequence: Synthetic peptide
<400> 32
   Cys Cys Cys Cys Arg Lys Gln Trp Lys Glu Thr Cys Cys Cys Cys Asn
   1 5 10 15
<210> 33
   <211> 17
   <212> PRT
   <213> Artificial Sequence
<220>
   <223> Description of Artificial Sequence: Synthetic peptide
<400> 33
   Cys Cys Cys Cys Thr Glu Asp Trp Lys Gln Arg Cys Cys Cys Cys Pro
   1 5 10 15
   Asn
<210> 34
   <211> 8
   <212> PRT
   <213> Artificial Sequence
<220> <223> Description of Artificial Sequence: Synthetic peptide
<400> 34
   Pro Cys Arg Lys Gln Asn Lys Trp
   1 5
<210> 35
   <211> 11
   <212> PRT
   <213> Artificial Sequence
<220>
   <223> Description of Artificial Sequence: Synthetic peptide
<400> 35
   Asp Trp Glu Thr Cys Cys Arg Lys Gln Trp Lys
   1 5 10
<210> 36
   <211> 16
   <212> PRT
   <213> Artificial Sequence
<220>
   <223> Description of Artificial Sequence: Synthetic peptide
<400> 36
   Lys Glu Thr Asn Cys Cys Arg Lys Glu Thr Asn Cys Cys Arg Lys Glu
   1 5 10 15
<210> 37
   <211> 8
   <212> PRT
   <213> Artificial Sequence
<220>
   <223> Description of Artificial Sequence: Synthetic peptide
<400> 37
   Thr Asn Cys Cys Arg Lys Glu Thr
   1 5
<210> 38
   <211> 8
   <212> PRT
   <213> Artificial Sequence
<220>
   <223> Description of Artificial Sequence: Synthetic peptide
<400> 38
   Asn Cys Arg Thr Glu Lys Gln Trp
   1 5
<210> 39
   <211> 9
   <212> PRT
   <213> Artificial Sequence
<220>
   <223> Description of Artificial Sequence: Synthetic peptide
<400> 39
   Asn Pro Cys Arg Thr Glu Lys Gln Trp
   1 5
<210> 40
   <211> 9
   <212> PRT
   <213> Artificial Sequence
<220>
   <223> Description of Artificial Sequence: Synthetic peptide
<400> 40
   Asn Thr Cys Arg Thr Glu Lys Gln Trp
   1 5
<210> 41
   <211> 10
   <212> PRT
   <213> Artificial Sequence
<220>
   <223> Description of Artificial Sequence: Synthetic peptide
<400> 41
   Asn Cys Cys Arg Thr Glu Lys Gln Lys Trp
   1 5 10
<210> 42
   <211> 9
   <212> PRT
   <213> Artificial Sequence
<220>
   <223> Description of Artificial Sequence: Synthetic peptide
<400> 42
   Cys Cys Arg Thr Glu Lys Gln Lys Trp
   1 5
<210> 43
   <211> 9
   <212> PRT
   <213> Artificial Sequence
<220>
   <223> Description of Artificial Sequence: Synthetic peptide
<400> 43
   Gln Trp Lys Glu Arg Thr Cys Cys Asn
   1 5
<210> 44
   <211> 8
   <212> PRT
   <213> Artificial Sequence
<220>
   <223> Description of Artificial Sequence: Synthetic peptide
<400> 44
   Lys Trp Glu Arg Cys Cys Arg Lys
   1 5
<210> 45
   <211> 13
   <212> PRT
   <213> Artificial Sequence
<220>
   <223> Description of Artificial Sequence: Synthetic peptide
<400> 45
   Gln Lys Arg Cys Cys Asn Thr Glu Asp Trp Ile Met Gln
   1 5 10

## Description

### TECHNICAL FIELD

The present invention relates to the field of immunology and peptide synthesis. More specifically, the disclosed invention relates to methods for designing and configuring surface simulation synthetic peptides incorporating amino acid sequences that simulate the 3 dimensional spatial position of non-sequential conserved residues necessary for viral pathogenicity. The disclosed surface simulation synthetic peptides are especially useful for incorporation into vaccines effective in eliciting an effective broad spectrum immune response against HIV-1, a hyper-variable viral pathogen.

### BACKGROUND OF THE ART

The immune system is comprised of a complex group of sub-systems which generally function through the identification and destruction/elimination of foreign antigenic materials which penetrate beyond the protection of natural physical barriers.

Specific antigen immunity is largely dependent upon presentation, memorization and identification of an amino acid sequence located upon an exposed/accessible portion of an antigen peptide. Generally, the target is a linear sequence of amino acids present in an antigenic peptide fragment which provides the situs of immune system activity.

A "hyper-variable virus", as the term is utilized throughout this specification and within the claims, refers to a viral pathogen which, due to substantial and continuing genetic mutation, exhibits substantial and continual variation in the amino acid sequences of the peptide and protein structures thereof while, at the same time, continuing to exhibit pathogenicity Such hyper-variable viral pathogens do present exposed and available sites for immune system recognition and memorization. However, due to the above-described high mutation rate -- and the continual amino acid variation caused thereby-- such immune system recognition and memorization is either greatly hindered or rendered completely ineffective. More particularly, although a subject viral protein residue sequence may be effectively recognized and memorized by the immune system, by the time antibodies are generated in response to initial recognition, the targeted sequence will often no longer exist in the subject pathogenic viral peptide fragment so as to allow such antibodies to function in an effector phase. HIV-1 is an excellent example of a hyper-variable pathogenic virus. It is known that a causative factor for high mutation rates demonstrated by HIV-1 lies in the failure of the reverse transcriptase therein to demonstrate proof reading functions. Thus, HIV-1 allows an inordinate amount of transcription errors to be passed along during replication. Such transcription errors, in turn, are responsible for causing constant variations in the amino acid sequence of the viral peptides defined thereby.

Despite HIV-1 sequence variations, the resulting new strains still manage to achieve successful infection of human cells. Although the immune system does mount responses against the virus, these responses are limited by the fact they are only strain-specific. The HIV virus mutates faster than the immune system can respond. In so doing the virus overwhelms and defeats the immune system. Other hyper-variable viral pathogens that evade the immune system in a like manner include the influenza virus and Hepatitis C.

HIV-1 is the agent responsible for AIDS. As a hyper-variable virus, HIV-1 incorporates proteins, key to viral pathogenicity, largely comprised of continually changing linear amino acid sequences. For example, gp120 is an envelope protein providing HIV-1 with key functions required for viral pathogenicity such as the ability to bind to the CD4+ T lymphocyte and thereafter allow the viral RNA to enter a host T cell. In addition, soluble gp120, apart from HIV-1, inhibits both the induction and effector functions of T-lymphocytes. In fact, soluble gp-120 is known to cause apoptosis of uninfected T-cells. Thus, gp120 is a key and required viral protein necessary for viral pathogenicity and, but for the highly variable amino acids therein, would present a most effective target for immune system intervention. Since gp120 is now well known to be the prime target of naturally occurring neutralizing antibodies, this glycoprotein, in particular, has been the subject of intense investigation . (ref. 1 - the scientific publication references herein are listed in the "References" section at the end of the specification)

Like all HIV-1 proteins, gp120 is subject to a wide array of mutations. In fact, HIV-1 strains are classified into clads, based on common variations encountered in the gp120 molecules. Within each clad, myriads of mutations continue to occur. Hence the challenge of mounting a successful immune response with cross-clad coverage presents an insurmountable task for the immune system unless all the HIV strains and clads can be reduced to some common denominators. As further explained below, SSSP's allow us to define such common denominators in the 3-D configurations of hyper-variable viral proteins such as gp120.

As mentioned above, the immune system ordinarily targets linear sequences within antigen peptide fragments. This process of antigen recognition does not, for the most part, take into account the 3-dimensional configuration of viral proteins which are, of course, critical for many protein binding functions. More specifically, antigen recognition and targeting largely depends upon presentation of a continuous linear sequence of a relatively small number of amino acids and does not generally involve recognition of discontinuous epitopes comprised of spatially proximate, but non-sequential residues. The term "non sequential conserved residues" and non sequential epitopes", as utilized throughout this specification and within the claims, refers to amino acid residues that are neither proximal or adjacent within the linear molecular arrangement of a subject peptide, but are related, spatially, in regards to 3 dimensional proximity as dictated by the proper folding of the proteins linear sequence. The term "proper folding" as utilized throughout this specification and claims refers to the three dimensional molecular configuration demonstrated by a subject protein and necessary in order to provide protein functions such as, for example, binding to a receptor site. Since most antigenic viral proteins maintain relatively consistent conserved residues -as consecutive elements of linear sequences-, immune system memorization of small fragments - comprised of as little as 8 to 10 amino acids- ordinarily provides sufficient information for later recognition, presentation and effector functions (when not presented with a hyper-variable virus/viral protein.

Viral proteins demonstrating a high rate of change within their linear amino acid sequences -as the result of increased viral mutation rates discussed above- fail to provide the host immune system with consistent linear epitope targets. As mentioned above, the virus responsible for human Acquired Immune Deficiency Syndrome, HIV-1, demonstrates very high mutation rate due to the fact that the reverse transcriptase produced by this organism lacks an effective "proof reading" function. Therefore, the resultant transcription errors inevitably lead to viral proteins demonstrating inconsistent amino acid composition. Thus, HIV-1 proteins, such as gp120, are not expected to, and do not present consistent linear epitopes. In fact, the linear amino acid sequence of gp120 is not only variable between different isolates, but is also highly variable between sequential isolates taken from a single infected patient. Yet gp120, notwithstanding such linear sequence variation, is able to continue demonstrating key protein functions (e.g., CD-4 binding). Therefore, it is not surprising, and it is now well known, that many of the functional binding sites of this key protein necessary for pathogenicity, are comprised of non-linear amino acid sequences that are approximated in the 3-dimensional configuration based upon proper folding of the molecule. Accordingly, the majority of effective, naturally occurring gp120 mab's with cross-clad coverage recognize discontinuous, non-sequential or "conformational" epitopes. (ref. 2 & 3) For example, it is know that the CD4+ binding site of gp120, a site, which, in conjunction with the CCR5-binding site, is required for viral entry into a host CD4+ T lymphocyte, is comprised of non-sequential epitopes consisting of several discontinuous residues (ref. 4) In fact, Thr257, Asp368, Glu370, Trp427, Asp457 -residues known to be conserved and required for CD4 binding- comprise a non-sequential epitope. (ref. 4)

It is also known that epitopes comprised of non-sequential conserved residues are successfully targeted and successfully bind with antibodies. For example, it is known that broad spectrum neutralizing antibodies to gp120 bind to such non-linear (also know as discontinuous) epitopes. (ref. 12)

As stated above, the immune system generally recognizes antigens via conserved linear residue sequences within small peptide fragments. Therefore it is not surprising that, in the past, peptides synthesized for the purpose of preparing antibodies -in both passive and active immunity development- have been similarly based upon the linear sequences of naturally occurring linear epitopes. However, it has also been known, to a much lesser degree, to utilize non-linear synthetic peptides as analogues to native sequences in 3-D configuration. More specifically, surface-simulation synthetic peptides "SSSP's" have been designed and engineered to simulate the 3-dimensional proximity of the amino acids constituents of large proteins. (ref. 15)
Such SSSP's have been known to successfully sensitize the immune system to recognize both the native protein upon which such synthetic peptides were patterned as well as the SSSP analogues. However, immune cells sensitized with such SSSP technology do not, in fact, complex with denatured target protein (Ref. 14). In such denatured proteins, sulphur bonds required to maintain a particular peptide conformation -and to achieve a necessary 3 dimensional spatial relation among the residues thereamong, are destroyed. Thus, SSSP's function by sensitizing the immune system to epitopes comprised of residues in a fixed 3 dimensional spatial relation, and not to linear sequences. SSSP's literally translate the surface 3D configuration into linear sequences to prime the immune systems. Since the immune system is apt to process linear sequences, SSSP's provide the most reliable strategy to allow the immune system to process and recognize discontinuous epitopes and to mount immune responses to the 3-D configurations of targeted proteins. Thus it is believed that SSSP strategies are and will prove necessary in the development of vaccines for unusual pathologies like 'Mad Cow Disease' and scrapie where the normal protein PrPc folds in a different (abnormal) 3-D configuration PrPsc in the presence of the prion entity.
As discussed below, the conserved entity of hyper-variable viral pathogens like HIV-1 lies in the 3-D configurations of the molecules and not in their linear sequences.

Even though hyper-variable viral pathogens may present protein constituents -such as gp120- lacking conserved linear epitopes, such proteins do maintain non-sequential conserved residues necessary for fundamental viral pathogenicity. These conserved residues are only approximated in the 3-dimensional configuration after proper folding of the protein. For example, in addition to conserved residues of the CD4+ binding site of gp-120 (required for binding and entrance into a host T-lymphocyte), an identified CC-CKR5 binding site is also required for viral entry and HIV binding. It is known that, in order for the CC-CKR5 site to function, conserved residues Arg419, Lys421, and Gln422 must be present. The conserved residues of the CC-CKR5 site lie, in turn, between two highy conserved residues: Cys418, (which is needed to maintain proper architecture in a disulfide bond with Cys385); and Trp427 which is required for CD4 binding. This segment of the gp-120 molecule -C418 to W427- is also of great interest because of this segment's ability to exhibit the "flip/switch phenomenon whereby the segment can simultaneously change from alpha-helix to beta-sheet configurations without passing through an intermediate conformation. (ref. 5) The fact that switch inhibitors", which are molecules designed to stabilize this segment in one configuration, thereby preventing the conformational change of the bistable flip/switch, are shown to also inhibit CD4-binding, strongly indicates that the bistable flip/switch phenomenon is important for CD4-binding. (ref.16) In order for HIV-1 to demonstrate infectivity, residue Asn262 must likewise be conserved.

It is known that conserved residues C385 and C418 are necessary for maintaining the structural integrity of gp120 by forming a disulfide bridge. As discussed in greater detail below, these cysteine residues have now been found to be required in order to maintain a unique tetrasulfide bridge which maintains a critical required 3 dimensional architecture without which gp120 can neither bind with or enter a host CD4+ T lymphocyte. C418 actually participates in the formation of the unique tetrasulfide bridge which maintains a critically required 3 dimensional architecture. Numerous studies have demonstrated the critical necessity of these cysteine residues to be present in the gp120 molecule in order to achieve functions such as CD4-binding, syncitium formation and infectivity. Thus, although highly variable viral proteins such as gp120 may be devoid of linear conserved epitopes, such molecules must and do retain certain residues, conserved in a 3-dimensional relation, in order to maintain viral viability.

As discussed above, the high mutation rate of HIV-1 results to changes in viral protein composition - the amino acid sequence thereof- at a correspondingly high rate. In addition, gp120 intercalates redundant non-immunogenic isoleucine residues among residues that are conserved despite such mutation rate so as to further blind the immune system. Therefore, the immune system is presented with very few conserved residues, many of which are partially or completely masked. Adding to these difficulties, the gp120 molecule is a highly flexible glycoprotein. Due to such flexibility, the 3 dimensionally conserved epitopes described above, are also in a state of spatial flux. However, it is also known that, like virtually all other proteins, in order to function -e.g., in order to bind at the CD4 site, accomplish CCR5 binding, enable flip switch operation- and thus enter a host cell and demonstrate infectivity-- the conserved residues of gp120 must assume a specific 3 dimensional configuration. Thus, the conserved entity of the HIV-1 lies primarily in the 3-dimensional configuration of its molecules and not in their linear sequences.

Given that highly variable viral pathogens, such as HIV-1, maintain conserved residues in a 3-dimensional configuration, it would be highly beneficial to obtain x ray crystallographic studies of key viral proteins. However, imaging of viral proteins, such as the highly flexible gp120 is difficult due to the constant changes in protein conformation. However, such imaging is possible when the subject protein has formed a complex with a receptor site located upon a target protein. For example, x ray crystallographic imaging has been obtained of gp 120 in complex with CD4 and a neutralizing antibody. (ref. 6)

It would be highly beneficial if a method could be devised for the identification, design and preparation of peptides especially suited for incorporation in vaccines effective in evoking an immune response against hyper-variable viral pathogens such as HIV-1 which do not present consistent linear amino acid epitopes, but do present non-sequential residues conserved in regard to their 3 dimensional conformation. It would be further beneficial to disclose peptides especially configured and adapted to evoke such an effective immune response. It would be still further beneficial if such peptides could be incorporated into immunologic reagents useful for the detection of hyper-variable viral pathogens.

### SUMMARY OF THE INVENTION

Now, in accordance with the present invention, a method as defined in claim 1 is disclosed whereby synthetic peptides, especially configured and sequenced In order to evoke an effective immune response with broad spectrum, including cross-clad coverage against hyper variable viral pathogens, are provided. More particularly, the method of the present invention is directed at the design and synthesis of surface simulation synthetic peptides highly useful for incorporation into vaccines effective in the prevention and treatment of HIV-1, a hyper-variable pathogen.

In the method of the present invention, a viral protein component, necessary and required for viral pathogenicity, is identified. Thereafter, non-sequential conserved residues therewithin, which are necessary and required for said viral pathogenicity, are identified. The subject protein is complexed with a natural binding site. Upon formation of the viral protein epitope/binding site complex, the subject viral protein is subjected to an imaging scan, such as, for example, x-ray crystallography, so as to obtain and record image data related to the 3 dimensional molecular configuration of the complexed protein. The 3 dimensional molecular image data obtained by said imaging scan is then subjected to computer analysis via a 3 dimensional protein analysis and rendering program in order to identify and obtain the 3 dimensional spatial relationships of said conserved residues. Upon identification of the spatial relationships of said non-sequential conserved residues, said software is utilized to configure surface simulation synthetic peptides (SSSP's) which incorporate conserved amino acid sequenced in accordance with the 3 dimensional proximity and orientation of said identified non-sequential conserved residues. The configured SSSP's are then further subjected to further protein computer software analysis so as to enhance the antigenicity thereof.

The term "viral protein necessary for viral pathogenicity" as used in throughout this specification refers to a protein required for key viral functions such as viral binding and infectivity. For example, gp120 is required in order for HIV-1 to enter a T lymphocyte. More specifically, the CD4 binding site of gp120 must, in fact, bind with a CD4+ T lymphocyte in order to initiate host cell entry. More importantly all gp120 molecules must conserve some key residues that are necessary in order to achieve the key functions such as CD4-binding, CCR5-binding and infectivity. Simply put, viral protein selection is based upon the fact that, regardless of strain or clad, all pathogenic viral pathogens require certain proteins with a number of conserved residues without which pathogenicity is not possible.

In practicing the method of the present invention, non-sequential conserved residues of viral proteins -which comprise 3 dimensional epitopes necessary and required for viral pathogenicity- are first identified. If the identity of such residues is not known, the subject protein is subjected to mutagenicity testing (site-directed mutagenesis studies) so as to identify such 3 dimensional epitopes comprised of non-sequential conserved residues. The term "necessary and required for said viral pathogenicity" refers to those 3 dimensionally conserved amino acid epitopes, without which, the subject protein can not provide the above-described pathogenic functions. For example, in regard to gp 120, site-directed mutagenesis studies have shown that Thr257, Asp368, Glu370, Trp427 and Asp457 are conserved residues necessary for CD4 binding. (ref. 4) Similarly, gp 120 has been shown to require the conserved residues Arg419, Lys421 and Gln422 for CC-CkR5 binding. Asn262 appears to be necessary for HIV-1 infectivity (ref. 7) It has also been shown that gp 120 requires Met 434 for infectivity. Further, and as to gp120, it is known that Cys385, Cys418, Cys331, Cys296 and Cys418 are conserved residues absolutely essential for maintaining protein geometry, architecture and function. In the absence of these conserved residues, the 3 D relationship required between the gp120 epitopes comprised thereof and CD4 binding sites and necessary for viral binding and host cell entry simply can not be attained. In addition these cysteine residues have now been discovered to also form a unique tetra-sulphide bridge, ("TTSB" )discussed in greater detail below, necessary for required protein binding with the CD4 site. Thus, the method of the present invention requires identification of conserved, non-sequential residues which are absolutely required and necessary for the subject virus to carry out special functions for the multistep process of infectivity.

After the non-sequential conserved residues have been determined, the subject protein is complexed with a natural binding site, (as well, in some cases discussed below, with a neutralizing antibody therefore). Binding provides stabilization of protein architecture which may, in the absence of such binding, be far too flexible for x-ray crystallography or other molecular imaging techniques. For example, gp120 is a highly flexible protein. X-ray crystallography has heretofore only been possible by means of first stabilizing the structure of gp-120 by means of complexing the glycoprotein with a CD receptor and a neutralizing antibody. (ref. 6) Most importantly, such binding provides the added advantage of providing a map of the exact conformation achieved by the conserved epitopes during binding -the spatial relationships and geometry necessary to provide epitope function and without which viral pathogenicity does not exist-.

In practicing the method of the present invention, upon formation of the viral protein/host binding site complex, the subject protein is subjected to an imaging scan such as, for example, x ray crystallography. The resultant image provides information regarding the 3 dimensional spatial relationships among the non-linear/non-sequential conserved residues based on proper folding of the protein in question. The term "3 dimensional spatial relationship", as utilized throughout this specification and claims, refers to the spatial relationship among conserved residues of a subject viral protein epitope necessary and required for required key viral protein functions such as, for instance, binding with a host cell receptor site. Thus, imaging data can be utilized to discover the intervening distances and angular relationships amongst the above-described conserved residues in 3-D. As discussed below, although non-sequential conserved residues may be separated by numerous intervening non-conserved amino acid residues, they nevertheless may be located within just a few Angstroms of one another spatially in the 3 dimensional view. Thus, the conserved residues may and, as discussed below, do form epitopes which are conserved, not as linear sequences, but in 3 dimensional proximity required for epitope function. The key to understanding the use of SSSP's is the fact that proteins are made as linear chains. Each linear chain folds in a certain way to arrange each link or amino acid in a specific 3-D configuration. After proper folding of a protein, some residues that are distant in the linear sequence (e.g. T257 and E370 in the gp120) may actually be approximated to less than 10 angstroms away from each other in the 3-D configuration.

The method of the present invention utilizes protein analysis software for both 3 dimensional functional mapping of residue conformation as well as to provide optimum configuration of SSSP's derived thereby. In the first instance, and as described in greater detail below, x ray crystallography data is analyzed utilizing such software in order to determine the identity and location, in 3 dimensions, of the amino acid constituents of the subject protein. Such computer analysis of the 3-D structure of the protein allows one to determine the positions of each of the selected non-sequential conserved residues. Functional maps of key residues generated by such software analysis of image data provide measurement of bond length, bond angles and distances between residues so as to provide true 3-dimensional rendering. The spatial proximity of the non-sequential conserved residues identified by such program analysis is then utilized to configure SSSP's which "mimic" the spatial proximity and proper 3-D arrangement of the conserved residues as determined by the functional map while ignoring the variable amino acid residues that often separate such residues in regard to their linear arrangement along the molecular chain. Thereafter, the SSSP's formulated are subject to further software analysis, as described in more detail below, in order to maximize sequence antigenicity, availability (hydrophobic/hydrophilic), remove non-antigenic amino acids and generally increase the efficiency of the SSSP in evoking an adequate immune response.

As discussed above, the methods and SSSP's of the present invention are highly useful in the diagnosis, treatment and prevention of hyper-variable viral pathogens. Although such viral pathogens may be otherwise quite disparate, it is well known that all such pathogens must produce viral proteins capable of binding with, and allowing entry into host cells- Since such viral protein functions require the production of conserved residues which bind with host cells in a particular 3 dimensional configuration, the methods of the present invention are applicable to any of such viral pathogens.

The SSSP's provided in accordance with the above and below described methods are useful in diagnostic reagents and kits incorporating such peptides. Since SSSP's generate peptides that may or may not exist in nature, it is necessary to screen each SSSP for homology with any known protein The purpose of this important step is to rule out possible cross-reaction with any known host's protein If such a cross-reaction occurs with a SSSP based vaccine then two possibilities may result: a) the host's immune system may be tolerant and recognize the SSSP segment as "self" and not mount any immune response against it or b) the immune response mounted against such SSSP with homology to the host's protein may lead to autoimmune diseases in the said host.

### Brief Description of the Drawings.

Fig. 1 is a computer generated image depicting the 3 dimensional spatial relationships of gp120 residues proven conserved and necessary for HIV-1 pathogenicity,
Fig. 2 is a computer generated image depicting, in particular, the conserved residues of gp120 cysteine residues necessary for the maintenance of vital tetrasulfide and disulfide bridges.
Fig. 3 is a computer generated image depicting, from a different spatial perspective, the cysteine bridges illustrated in Fig. 2 and a tetrasulfide bridge necessary for viral pathogenicity.

### Detailed Description

As discussed above, HIV-1 is the highly mutating pathogenic virus causative of acquired immunodeficiency syndrome -AIDS-. The HIV-1 envelope protein, gp 120, is critical to the pathogenicity of HIV-1 due to the protein's inclusion of conserved residues determined by site directed mutagenesis studies to be necessary for binding and entry into a host T cell. For example, gp120 demonstrates a CD4 binding site comprised of conserved residues Thr257, Asp368, Glu370, Trp427 and Asp457) and a CC-CRK5 binding site comprised of conserved residues Arg419, Lys421 and Gln422 both of which define epitopes required for viral binding and entry into a host CD4+ T lymphocyte. Asn262 is also a conserved residue of gp120 that is necessary for HIV-1 infectivity. In addition, gp120 includes cysteine residues Cys385, Cys418, Cys331, Cys296 and Cys418 as non-sequential conserved residues necessary for maintaining a 3 dimensional gp 120 conformation critical to the key pathogenic functions of gp120 including viral binding and infectivity. The residues required for CC-CKR5 binding lie within a segment of gp120 located between two highly conserved residues, Cys418 (required for maintaining critical 3 D architecture of gp120) and Trp427 which is required for CD4 binding. The subject segment is, as described above, also highly critical in that the segment between subject residues C418 - W427 demonstrate the bistable flip/switch phenomenon which is also required for CD4 binding. This segment is highly conserved with 5 necessary residues Cys418, Arg419, Lys421,Gln422 and Trp427 out of 9 total residues (from 418 to 427). While highly conserved, this segment is rendered non-immunogenic by the many isoleucine residues intercalated between the necessary residues. The Isoleucine residues actually provide stealth and render the conserved segment invisible to the immune system.

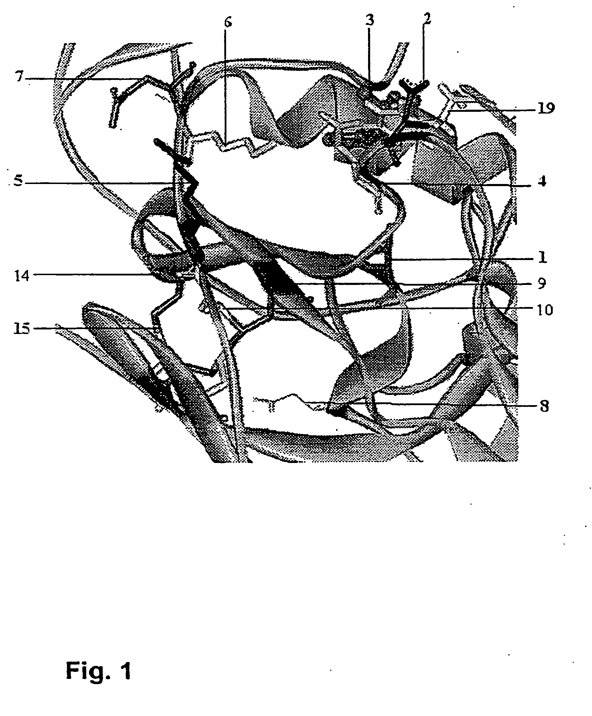

**Fig. 1** is a computer generated model of gp120 resulting from analysis of a gp120 molecule binding to a CD4 receptor site. Each of the numbers listed in Fig. 1 refers to a residue conserved in a 3 dimensional relationship with other such residues. The residues illustrated in **Fig. 1** are each necessary and required for a key protein function. For example, T-257 (**1**), D-368 (**2**), W-427 (**3**) *(represented in sticks and balls),* E370 (**4**) and D-457 (not illustrated in this view) define an epitope necessary for CD4 binding. F43 (**19**) *(Phenylalanine 43)* is a key residue in the CD4 molecule which interacts with the gp120 of HIV-1. Residues necessary for CC-CKR5 binding are R-419 (**5**), K-421 (**6**), and Q-422(**7**). N-262 (**8**) is required for infectivity. In addition, C-385 (**9**) and C-418 (**10**), necessary for an architecturally critical di- and tetrasulfide bridge discussed below, are also illustrated. Although each of the afore-mentioned residues may be separated from each other by numerous amino acids -when viewed as linear elements of gp120 -each of the afore-mentioned conserved residues is no more than 10 Angstroms from its most proximate 3 dimensionally conserved neighbor residue.

The present invention specifically targets the CD4-binding side of the gp120 molecule as opposed to any other part of the molecule. As described in detail below, this is a strategic plan designed to neutralize all potential immunoregulatory effects of the soluble form of gp120. In the past, it has been postulated that the recessed nature of the CD4-binding pocket of the gp120 may limit the generation and effect of high-affinity antibodies to the CD4-binding site. In fact, studies on antibodies that show broad-spectrum neutralization of various HIV strains reveal that these antibodies actually have epitopes that include residues that are located in the CD4-binding pocket and were later found to be necessary for CD4 binding. (ref. 12) In view of the immunomodulating properties of the gp120 molecule, the present invention advantageously and specifically targets the CD4-binding side of the gp120 for the following reasons:
i. There is mounting evidence of the immunosuppressive effects of HIV-1 gp120. Soluble gp120 inhibits T-cell-dependent B cell differentiation both at the inductive phase of T-cell activation and at the effector phase. (ref. 9) Soluble gp120 causes apoptosis in uninfected CD4+ cells. Humoral response to HIV gp120 can sensitize non-infected cells towards apoptosis and contributes to T cell decline upon administration of recombinant gp120. (ref. 10)
ii. If an HIV vaccine target is located on the opposite side of the CD4-binding site of gp120 (or on any side otherthan the CD4-binding side), then soluble gp120 molecules are still allowed to bind to uninfected CD4 cells while exposing their targeted side to immune attack, which may inadvertently cause the destruction of the uninfected CD4 cells (as innocent bystander) by the immune system.
iii. Targeting the CD4-binding side as opposed to any other exposed side of the gp120 molecule will prevent immune-mediated destruction of uninfected CD4+ cells by the self-defeating mechanism described above in scenario "ii".
iv. The key residues targeted for the SSSP's of the present invention encompass an area between the CD4-binding residues and the CCR5-binding residues and the TTSB (tetrasulfide bridge). As mentioned earlier the CCR5-binding segment also participates in the bistable flip/switch phenomenon, which is necessary for CD4-binding.

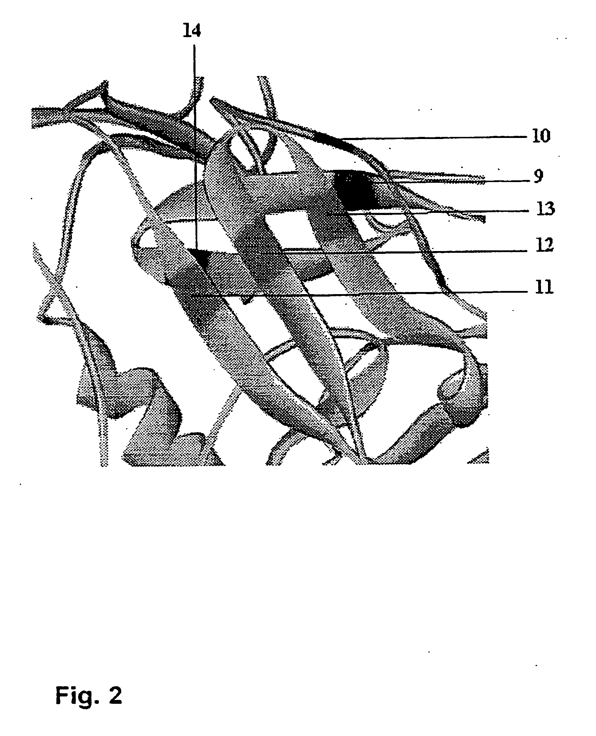

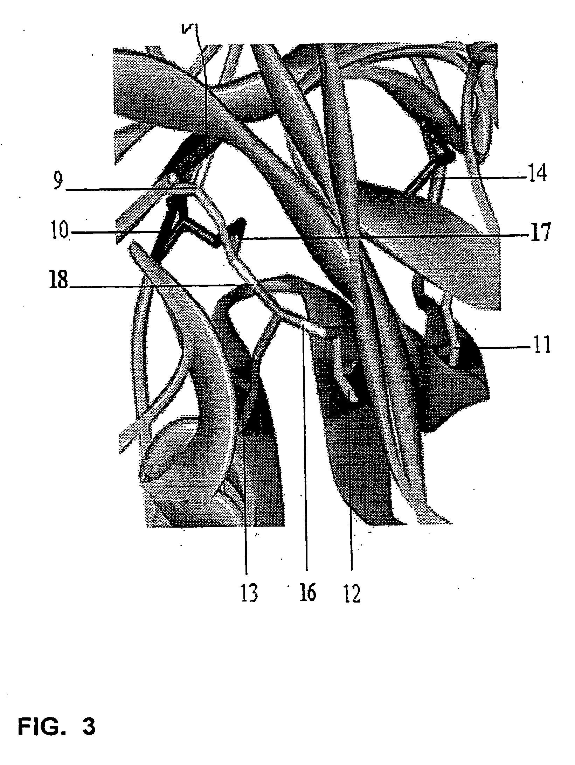

As mentioned above, it has now been discovered that Cys385, Cys418, Cys331, Cys296 and Cys418 are conserved residues absolutely essential for maintaining protein geometry and function. Without these conserved residues, the 3-D relationship required between the gp120 epitopes and T cell CD4 binding sites necessary for viral binding and host cell entry simply can not be attained. **Fig. 2** is a computer generated image representing the outer domain of the subject CD4 bound gp120 molecule. (ref. 6) The image includes a beta-pleated sheet with three strands spanning the central area. Each of these strands contains a cysteine residue. These Cysteine residues are actually juxtaposed in the same plane in the 3-dimensional configuration. In **Fig. 2** ,as well as **Fig. 3**, these Cysteine residues are, from left to right: Cys445 (**11**), Cys296 (**12**) and Cys331(**13**). In another plane, behind these 3 juxtaposed cysteine residues, lies another Beta sheet that runs almost perpendicular to the first one. There are 2 other cysteine residues in this second sheet: Cys385(9) and Cys378(**14**) (also shown in **Fig. 1**). Then, on the side, between Cys331 (**13**) and Cys 385(**9**), runs another string of amino acids which contains Cys418(**10**).

As discussed above, it has heretofore been known that gp120 demonstrates disulfide bridges necessary and required in order to obtain the 3 dimensional configuration that allows viral binding to the CD4 + T lymphocyte. Comprising these critical architectural features:
1. Cys378 makes a disulfide bridge with Cys445 (15);
2. Cys296 makes a disulfide bridge with Cys331 (16); and
3 Cys385 makes a disulfide bridge with Cys418 (17).

Fig. 3 illustrates the above-described disulfide bridges from a different orientations so as to reveal the following. It has now been discovered that from bridge "16" to bridge "17", the sulfur atom from Cys331 shares another bond with the sulfur atom of Cys385 forming a new connection (CYS331:SG - CYS385:SG (18)) that links the CYS296:SG - CYS331:SG bridge (16) with the CYS385:SG - CYS418:SG bridge (17). Thus the CYS331:SG - CYS385:SG (**18**) forms a bond between two disulfide bridges to form a newly discovered tetrasulfide bridge or TTSB.

While TTSB's may be discovered to exist in other proteins, this CYS331:SG - CYS385:SG bond (TTSB) represents a unique landmark in the architecture of the gp120 of HIV-1. It is well known that disulfide bridges add structural integrity to protein structures. It is believed that the tetrasulfide bridge further aids such structural integrity via the additional cross-linking of disulfide stabilized protein segments. It is known that, despite the hyper-variable nature of HIV-1, and the resultant variations in gp120 sequence composition, the glycoprotein manages to maintain the afore-mentioned 3 dimensional structure necessary for required viral function. Utilization of the tetrasulfide bridge is an efficient way for gp120 to utilize a minimum amount of non-sequential conserved residues to maintain such functionally necessary architecture. As demonstrated in the figures, two beta sheets in different planes are kept approximated by one disulfide bridge Fig. 1 **(15)** and one tetrasulfide bridge **(18).** In the absence of the tetrasulfide bridge, the alpha helix beginning at Ala336 and Arg335, just distal to Cys331 would not be maintained as such and, in fact, gp120 would not demonstrate the 3 dimensional configuration necessary for binding with, and entry into the CD4+ T lymphocyte -viral pathogenicity would not be maintained. In fact, the critical nature of the tetrasulfide bond is supported by the literature. Site-directed mutagenesis studies has proven that mutations at Cys296, 331, 418 and 445 resulted in noninfectious mutants whose gp160 envelope precursor polypeptide was poorly cleaved whereas mutations at Cys131 and Cys196 resulted in noninfectious mutants that were defective for syncytium formation with a late block in life cycle. (ref. 8) This same study also shows that viruses with mutations at Cys386 were still infective, but defective for syncitium formation. The Cysteine residues involved in the TTSB clearly have a key role in the pathogenicity of HIV-1 and comprise preferred conserved residues incorporated into the SSSP's of the present invention.

The HIV-1 virus has many unusual properties (e.g. inhibition of phagosome-lysosome fusion) that have thus far remained unexplained. Soluble gp120, in the absence of the virus proper, has its own independent immunosuppressive effects. For example, soluble gp120 inhibits T-cell-dependent B cell differentiation both at the inductive phase of T-cell activation and at the effector phase. (ref. 9) It is also well known that soluble gp120 causes apoptosis in uninfected CD4+ T cells. Humoral response to HIV-1 gp120 can sensitize non-infected cells towards apoptosis and contributes to T cell decline upon administration of recombinant gp120. (ref. 10) The specific mechanisms responsible for the above-described immunosuppressive effects of gp120 are largely unknown. Certainly, such effects have not been attributed to the disulfide bridge configuration. However, Gliotoxin and related epipolythiodioxopiperazines (ETP's) do form TTSB's in solution and they have similar in vitro immuno-modulating activities including apoptosis and inhibition of phagocytosis. These immunosuppressive effects have been attributed specifically to the sulphur bridges in these molecules. (ref. 11)

While practical for the virus, the TTSB also presents a complex fragility for the gp120, which provides one explanation as to why many antibodies that bind to the V3 loop (located between the Cys296 and Cys331 disulfide bridge involved in the TTSB) can easily prevent CD4-binding and neutralize HIV-1. Another complex fragility for the TTSB is the fact that this TTSB is attached, by way of Cys418, to the segment 416-427. As mentioned earlier this segment is involved in the flip/switch phenomenon which is necessary for CD4-binding. Moreover this segment [416-427] also contains the residues that are necessary for CC-CKR5 binding..

In addition to the conserved residues discussed above, some of the amino acid residues of gp120 are found to be redundant in certain localized areas of the glycoprotein. For example, Asn386 is redundant, but not necessarily conserved. Although many of these residues are not necessarily proven to be conserved individually, some can be considered as potential targets for immune response elicited by SSSP's in view of their 3-Dimensional proximity to some of the above-identified conserved residues. (See Table 1 (b), below.) For example, Thr373 and Thr388 are positioned only 2.86 and 13.38 Angstroms -respectively- from conserved residue Thr257. As another example Trp112 is located 4.82 Angstroms away from Trp427. The Ref. 6). It is critical to note that the subject x-ray crystallography was performed with gp120 binding to a CD4 receptor. Since, as described above, gp120 is a highly flexible glycoprotein, imaging of gp120 alone would be highly difficult if not impossible. More importantly, examination of gp120 bound to a CD 4+ receptor and antibodies reveals the 3 dimensional relationships of the above-described conserved residues which are required for such binding. The subject gp120 crystallized in this ternary structure was from the HXBc2 strain of HIV-1 (ref. 6). The PDB format of the file 1 GC1 was downloaded from the PDB website.

Various studies of the gp120 molecule, including the generation of functional maps of key residues and measurement of bond length, bond angles, distances between residues and measurement of bond angle were achieved using the Swiss PDB Viewers (versions 3.5 and 3.6 beta), Alchemy 2000 (version 2.05), SciProtein version 2.0) and WebLab ViewerPro (version 3.7) software packages. The Swiss PDB viewers were downloaded from the ExPASy Website. The Alchemy 2000 and the Sciprotein software packages were purchased from Tripos, Inc. and SciVision, Inc., respectively. The WebLab ViewPro was purchased from Molecular Simulations, Inc.
All computer-aided operations were performed on IBM-compatible personal computers running Windows 98. The generated graphics were either saved in BMP format by the respective software or captured in BMP format using the Pizzaz Plus software.

In addition to all of the afore-mentioned residues, there have been found to be many redundant Isoleucine residues that seem to agglomerate in certain areas, namely Ile423, Ile424, Ile109, Ile110 and Ile420. The above-described analysis of SSSP antigenicity utilizing the SciProtein software demonstrates that SSSP's containing Ile residues are far less immunogenic than those where Ile residues are strategically omitted. It is thus believed that the HIV-1 virus evades the immune system, in part, by incorporation such redundant Ile residues in proximity to the above-described 3 dimensionally conserved residues. Thus, in regards to the above-described mutation at Met434, wherein Isoleucine replaces Met434, the Met434 variant is favored for SSSP design based upon the relative antigenicity of the subject residues.

In accordance with a preferred embodiment of the present invention, SSSP's are designed based upon the spatial configuration of the afore-mentioned conserved residues as identified on functional maps of gp120 generated by means of the afore-mentioned Swiss PDB viewers, Alchemy 2000, SciProtein and WebLab software programs. More specifically, based upon the 3 dimensional spatial proximity of the subject conserved residues, SSSP sequences were designed to mimic such 3 dimensional proximity by ordering the SSSP's in accordance with the derived distance data. Thus, the SSSP's are designed so that each sequential amino acid incorporated within the synthetic molecule is representative of a native conserved amino acid which is spatially positioned, in 3-D, within the gp120 molecule, no more than 10 Angstroms from its most proximate, conserved native residue. Thus, although the SSSP's of the present invention do not represent linear native amino acid sequences, they do represent the 3 dimensional spatial relationships of the above-described conserved residues with respect to distance and orientation.

For SSSP strategies to be possible two requirements must be met:
i. Key residues of interest must be identified; and
ii. A 3-dimensional structure of the protein in question must be available.
Key residues can be identified by various means. Review of the literature may reveal importance of some residues in well studied proteins. The importance of residues on a target protein is best evaluated by site-directed mutagenesis studies (SDMS) However site-directed mutagenesis studies require prior knowledge of functions and properties of the said protein to begin with. Though SDMS's are costly and labor intensive projects, many are reported in the literature on important well studied proteins like the gp120 of HIV-1. SDMS identify conserved residues that bear key importance or functional significance for the said protein by testing outcomes for key functions after particular mutations are introduced to replace specific residues. Identification of conserved residues can also be achieved from alignment analyses of variant strains of the said target protein. The sequences for various strains of many important proteins are available on various databases on the Internet. Alignment analysis consists of putting the linear sequences of all the available variants of a target protein on top of each other in a way to match sequences based on common segments or residues.

### Resolution of 3-D configuration of target proteins

3-dimensional structures of proteins are best resolved by X-ray crystallography, a procedure that is well known by those skilled in the art. However to date, X-ray crystallography has many limitations and cannot resolve the 3-D structures of protein molecules that are either too small or too flexible. Many molecules of interest, such as the gp120 of HIV-1, fall into that category of molecules whose 3-D configurations cannot be resolved by standard X-ray crystallography. To resolve this limitation, two strategies can be used. As a first strategy, one may bind the protein of interest to a ligand or receptor if the ligand or receptor is known. Most importantly, such binding provides the added advantage of providing a map of the exact conformation achieved by the conserved epitopes during binding -the spatial relationships and geometry necessary to provide epitope function and without which viral pathogenicity would not exist. In this strategy it should also be noted that complete binding to a receptor may significantly alter the 3-D configuration of a target protein when compared to the unbound entity.

In a preferred embodiment of the present invention limitations of the afore-mentioned imaging strategy by what may be appropriately termed "Mosaic Crystallography". This presently disclosed method is especially advantageous in overcoming the limitations of standard crystallography where small and flexible proteins are concerned.

The purpose of the imaging protocol disclosed herein is to resolve 3-dimensional configurations of proteins that are too flexible and/or small to allow standard crystallography. With the advent of SSSP (Surface Simulation Synthetic Peptide) strategies, the 3-D configurations for proteins of interest become necessary challenges which must be resolved in order to allow completion of any projects based on SSSP's. Many molecules of interest including the HIV gp120 and many other flexible or small proteins (e.g. hypervariable viral glycoproteins, receptors, tumor markers) that have yet to be discovered, fall in the category of potential target for SSSP technology.
3-D configurations of small flexible proteins-can also be achieved by binding them to other stable ligands or receptors if available. However completion of binding to these ligands or receptors may markedly change the 3-D configuration of the unbound protein The protocol described below allows resolution of the 3-D configurations of such proteins to be achieved in shortest time frame.

Mosaic Crystallography is best used for protein structures which cannot be resolved by conventional crystallography. It is based on the fact that antibodies, being large molecules, allow good crystallographic pictures. While some proteins may be too flexible and/or small to allow standard crystallography, when bound to antibodies, the antibodies retain enough integrity to resolve the structure of the said target protein. When bound to proteins, pieces of the bound proteins actually remain attached to the antibody at the time of crystallography. Depending on the size of the remaining piece of the target protein attached to the antibody in the crystallographic picture, information on the tertiary and secondary structure of that portion of the target protein can be obtained.
I. Let us assume, for example, a small flexible protein called VP1 (for Viral Protein #1) with 5 regions or domains D1, D2, D3, D4, D5.
II. Given the linear sequence for VP1, various antibodies can be made against each domain separately. Label each batch of antibody according to the domain from which they were derived: AbD1, AbD2, AbD3, AbD4, AbD5.
III. When bound to the respective domain of VP1, the resulting structures are labeled accordingly: Ab*D1-VP1, Ab*D2-VP1, Ab*D3-VP1, Ab*D4-VP1, Ab*D5-VP1. This nomenclature denotes that the antibodies are bound to the respective domains while the said domain is still part of VP1. These structures are actually complexes of VP1 bound to antibodies; but the antibodies are bound to different domains.
IV If a set of antibody fails to bind to the native VP1, it implies that the domain for which it was made is not accessible on the surface and may be located in deeper planes of the VP1 structure.
V Based on protocols well known to those skilled in the art, perform crystallographic studies on each complex Ab*D1-VP1, Ab*D2-VP1, Ab*D3-VP1, Ab*D4-VP1, Ab*D5-VP1 separately.
VI Given the flexible nature of VP1, one should not expect whole structures of VP1 complexed with any one antibody. The result will likely yield antibodies complexed with the respective domains: Ab*D1, Ab*D2, Ab*D3, Ab*D4, Ab*D5 separately. Each domain is captured independently.
VII To resolve the 3-D configuration and more importantly the surface of VP1, we now need to solve the puzzle by constructing a mosaic of the independently captured domains based on a best-fit scenario. This result is termed a PRIMARY MOSAIC. Rearranging the structures: Ab*D1, Ab*D2, Ab*D3, Ab*D4, Ab*D5 in the primary mosaic will yield significant information about the surface of VP1.
VIII Confirmation and/or ambiguity in the primary mosaic can be resolved in the secondary mosaic as follows:
   a) Based on the primary mosaic, estimate which domain is closer to each other.
   b) Rank the domain in respect to proximity based on the primary mosaic
   c) React VP1 with all the domain antibodies. If a stable structure is achieved, label it: Ab*D(₁₋₅)-VP1, then go to step f) below.
   d) If no stable structure is achieved then react VP1 with one domain antibody (AbDn where n can be any number of the 5 domains) The result yields Ab*Dn-VP1. Save Ab*Dn-VP1 for the next step.
   e) React Ab*Dn-VP1 with the antibody domain closest to n based on the ranking process in step 8b. The purpose of this sequential addition of antibodies is to stabilize VP1 with antibodies while avoiding tearing the structure. Save each subsequent structure after adding antibodies to approximated domains. The binding site of some antibodies at this point may be blocked by a previously added antibody. It should be so noted if it is observed. Continue to add the next closest antibody. The resulting structure would be VP1 complexed with two or three or even more antibodies to preferably approximated domains. Appropriate nomenclature: Ab*Dn_x-VP1 where n_x denotes the antibody domain complex deemed to be in sequential proximity.
   f) Collect the Ab*Dn_x-VP1 (or Ab*D(₁₋₅)-VP1 if available) complex and perform crystallographic studies according to protocols well known to those skilled in the art. This step is termed SECONDARY MOSAIC crystallography. It yields the picture of large portions of the target protein, if not the whole protein, complexed to multiple antibodies.
   g) Analyze in 3-D rendering viewer software.

Mosaic crystallography, as the term is utilized throughout this specification and within the claims, is the composite 3-D picture of a protein resolved from crystallographic snapshots of various pieces of the said protein bound to various antibodies described above. It solves the 3-D configurations of proteins that are too small or too flexible for standard crystallography methods.

In accordance with the methods of the present invention, the 3 dimensional molecular image data obtained by a crystallographic imaging scan, including, in a preferred embodiment, mosaic crystallography, is then subjected to computer analysis via a 3 dimensional protein analysis and rendering programs in order to identify and obtain the 3 dimensional spatial relationships of said conserved residues. Upon identification of the spatial relationships of the afore-mentioned non-sequential conserved residues, the above-described analysis software is utilized to measure distances between conserved key residues and to configure surface simulation synthetic peptides which incorporate conserved or necessary amino acid sequenced in accordance with the 3 dimensional proximity of said identified non-sequential conserved residues. The configured SSSP's are then further subjected to additional protein computer software analysis so as to increase the antigenicity thereof.

After configuring SSSP's in accordance with the above-described method, each SSSP sequence is run through databases utilizing, for example, FASTA engines on the Internet in order to search for homology patterns. Each SSSP must be so tested in order to avoid cross-reactions with native human proteins that could result in autoimmune side effects from a vaccine derived from such SSSP's. Thereafter, each SSSP is evaluated, utilizing protein analysis software, such as, for example, the afore-mentioned SciProtein software, in order to evaluate SSSP antigenicity, hydropathy, accessibility and flexibility. The results of such testing are utilized in order to optimize the effectiveness of the subject SSSP's. For example, important residues such as Tryptophan 427, can have its relatively low antigenicity augmented by placing another key residue such as, for example, lysine, in its vicinity.

The present invention contemplates incorporation of multiple functional epitope analogs within the derived SSSP sequence. For example, the conserved residues necessary for CD4 binding -Trp427, Asp368, Glu370 and Thr257- and the conserved residues necessary for CC-CKR5 binding Arg419, Lys421 and Glen422- may be incorporated into the sequence of the same SSSP. The epitope represented by such SSSP's is derived in accordance with the 3 dimensional spatial proximity of the native conserved residues. The SSSP's described herein are represented by the international one-letter symbol system and/or three letter abbreviation for each amino acid residue therein as follows:

| | | |
|---|---|---|
| A | alanine | Ala |
| R | arginine | Arg |
| N | asparagine | Asn |
| D | aspartic acid | Asp |
| C | cysteine | Cys |
| Q | glutamine | Gln |
| E | (glutamic acid) | Glu |
| G | (glycine) | Gly |
| H | (histidine) | His |
| I | (isoleucine) | Ile |
| L | (leucine) | Leu |
| K | (lysine) | Lys |
| M | (methionine ) | Met |
| F | (phenylalanine) | Phe |
| P | (proline) | Pro |
| S | (serine) | Ser |
| T | (threonine) | Thr |
| W | (tryptophan) | Trp |
| Y | (tyrosine) | Tyr |
| V | (valine) | Val |

Referring to **Fig. 1**, one may design a "zipped like epitope analog incorporating the sequence QWKERTCcN (SEQ I.D. 43) following one spatially proximate order. On the other hand, one may also design a "V" shaped epitope incorporating an SSSP sequence of QKRCcTED. Furthermore, one can also design a "small circle" epitope represented by an SSSP sequence of KWERCcRK (SEQ I.D. 44) and two larger "circle" epitopes comprised of SSSP sequences of QKRCcTEDW and QKRCcnTEDWIMQ (SEQ I.D. 45). In selecting any such geometric epitope, the prime consideration is that the incorporated order should reflect the spatial proximity of sequential amino acids of the native epitope represented by the amino acid sequence of the synthetic analog thereof -the derived. Table 1, below, includes inter-residue distances between key conserved residues. The measurements listed therein are based upon the distance between the most proximate atoms of each respective residue derived from the functional maps of gp120 described above. Table 1(a) lists the distances measured between various nearby atoms in the respective non-sequential conserved residues and Table 1 (b) lists distances before the subject non-sequential conserved residues and the above-described redundant residues.

**TABLE 1(a)**

| Residues Examined | Distance (Angstroms) |
|---|---|
| Asp457 - Arg419 | 29 |
| Asp457 - Asp368 | 18.3 |
| Asp457(ODI) - Thr257(O) | 17.01 |
| Gln422 - Glu370 | 11 |
| Gln422 - Asp368 | 15 |
| Thr257 - Glu370 | 5.3 |
| Thr257(CG2) - Glu370(O) | 2.99 |
| Thr257(CG2) - Trp427(CE2) | 9.4 |
| Thr257(O) - Cys385(O) | 7.66 |
| Thr257 - Cys385 | 9.5 |
| Thr257 - Cys418 | 13.5 |
| Thr257 - Arg419 | 13.8 |
| Thr257 - Glu370 | 6.9 |
| Thr257 - Phe43 | 6.7 |
| Thr257 - Gln422 | 18 |
| Thr257 - Lys421 | 13.9 |
| Thr257 - Arg419 | 14.9 |
| Phe43 - Glu370 | 4.16 |
| Phe43 - Trp427 | 4.73 |
| Phe43 - Gln422 | 12.9 |
| Lys421 - Glu370(base) | 9.8 |
| Asp368 - Arg419 | 15.9 |
| Asp368(O) - Trp427 | 9.6 |
| Asp368 - Glu370(N) | 9.8 |
| Gln422 - Trp427 | 16.6 |
| Trp427 - Ser115 | 11.29 |
| Trp427 - Thr257 | 10 |
| Trp427 - Gln422 | 16.6 |
| Trp427(CB) - Glu370(CG) | 6.73 |
| Glu370 - Lys421 | 7.27 |
| Glu370 - Arg419 | 13.6 |
| Glu370 - Gln422 | 10.4 |
| Asn262 - Cys418 | 18.9 |
| Asn262 - Gln422 | 24.3 |
| Asn262 - Ser115 | 18.3 |
| Asn262 - Arg419 | 20.37 |
| Asn262 - Thr257 | 12.9 |

**TABLE 1(b)**

| Distances from conserved residues to redundant residues | |
|---|---|
| Residues Examined | Distance (Angstroms) |
| Trp427(NE1) - Trp112(NE1) | 4.82 |
| Trp427(CZ2) - Trp112(CZ3) | 5.20 |
| Thr257(CB) - Thr373(O) | 4.0 |
| Thr257(OGI) - Thr373(O) | 2.86 |
| Thr257(O) - Thr388(N) | 11.29 |
| Thr257(O) - Thr388(OGI) | 13.38 |
| Thr257(O) - Asn386(ODI) | 12.14 |
| Asn262(CB) - Asn386(O) | 19.36 |

| | |
|---|---|
| *(The letters in parentheses pertain to the atoms from which the measurements were made)* | |

SSSP's configured in accordance with the methods of the present invention are listed in Table 2. Within Table 2: "Cc" denotes two cysteine residues in a bridge. Lower case letters and shaded capital letters denoted residues that may be omitted in order to further optimize the SSSP's. Note thatAsn262, being in a different plane, may be a poor target in the subject SSSP's. It is noted as "N" on the list. While it is conserved, immune responses to this SSSP may not optimally respond to it as a potential target in view of the distance and 3-D orientation. However, there are a number of redundant Asn (e.g. Asn386), which may be targeted.

**TABLE II**

| SSSP | SEQ ID: NO: |
|---|---|
| DWETCNNCRKEDW | SEQ ID: NO:1 |
| NCRKEDWDEKRCN | SEQ ID: NO:2 |
| DWETCNCTEWD | SEQ ID: NO:3 |
| WDETCNCTEDWDETCN | SEQ ID: NO:4 |
| NCRTEKQWQKETRCN (Zipper 1) | SEQ ID: NO:5 |
| QWKERTCN | SEQ ID: NO:6 |
| QKWETCcRKW | SEQ ID: NO:7 |
| QKWDETCcRKW | SEQ ID: NO:8 |
| DWETCN | SEQ ID: NO:9 |
| DWETCCRKQWK (Large circle) | SEQ ID: NO:10 |
| QKRCcTEDW | SEQ ID: NO:11 |
| QKRCcnTEDWIIMQ | SEQ ID: NO:12 |
| QKRCcTED | SEQ ID: NO:13 |
| DWEKRCcnTEDWK | SEQ ID: NO:14 |
| QKRCcTED | SEQ ID:NO:15 |
| WQKRCcTEDW | SEQ ID: NO:16 |
| KWETCcRK | SEQ ID: NO:17 |
| KWETCcRKQ | SEQ ID:NO: 18 |
| TNCCRKET (Smaller circle) | SEQ ID: NO:19 |
| DWETNCCRKQWK | SEQ ID: NO:20 |
| IWDETNCCRKQIWK (With redundant residuals) | SEQ ID: NO:21 |
| IWDETNCCRKQNIWK (With redundant residuals, last N for mutant at Ser 115) | SEQ ID: NO:22 |
| LPCRKQMIW | SEQ ID: NO:23 |
| LPCRKQMIWDWETNC | SEQ ID: NO:24 |
| LPCRIKQMIW | SEQ ID: NO:25 |
| LPCRIKQMIWDWETNC | SEQ ID: NO:26 |
| LPCRKQWDWETCNc | SEQ ID: NO:27 |
| LPCcTEWD | SEQ ID: NO:28 |
| LPCcTEWKR | SEQ ID: NO:29 |
| LPCCCCRK | SEQ ID: NO:30 |
| CCCCRKWETCCCCN | SEQ ID: NO:31 |
| CCCCRKQWKETCCCCN | SEQ ID: NO:32 |
| CCCCTEDWKqRCcccPN | SEQ ID: NO:33 |
| PCRKQNKW | SEQ ID: NO:34 |
| DWETCCRKQWK | SEQ ID: NO:35 |
| KETNCCRKETNCCRKE | SEQ ID: NO:36 |
| TNCCRKET | SEQ ID: NO:37 |
| NCRTEKQW | SEQ ID: NO:38 |
| NPCRTEKQW | SEQ ID: NO:39 |
| NTCRTEKQW | SEQ ID: NO:40 |
| NCCRTEKQKW | SEQ ID: NO:41 |
| CCRTEKQKW | SEQ ID: NO:42 |

| | |
|---|---|
| **"Cc" denotes two cysteine residues in a bridge. Lower case and bold capital letters denote residues that may be omitted in order to further optimize the SSSP's.** | |

It is preferred that vaccines, based upon the peptides prepared in accordance with the methods of the present invention, be comprised of a polyvalent mixture of the SSSP's derived thereby For example, in regards to HIV, polyvalent mixtures of the SSSP's listed in Table 1 would increase vaccine efficacy. Furthermore, it is preferred that the SSSP's derived by means of the method of the present invention be arranged in various combinations of tandems, mirror images and tandem repeats in order to provide greater flexibility, evoke a greater antigenic response and thereby provide a more efficacious vaccine

Vaccines containing the peptides of the present invention may be prepared as liquid solutions, emulsions, injectables, suppositories and inhalation/pulmonary compositions. It is contemplated that said immunogenic compositions will include, in addition to said at least one SSSP'(s) or nucleic acid coding therefore, pharmaceutically-acceptable expedients such as, for example, water, saline, dextrose, glycerol, ethanol, and combinations thereof In addition, and particularly in regard to inhalation administration, the SSSP's of the present invention may be incorporated and carried by a surfactant composition useful and effective in such administration. Such immunogenic compositions may also include liposome carriers for the administration of said SSSP(s) and/or coding molecules.

The SSSP's of the present invention may be provided by nucleic acid molecules encoding therefore. Such molecules may be advantageously utilized in a direct method by administering the nucleic acid directly into the host system Alternatively, such encoding molecules may be incorporated into a live vector such as, for example, an adenovirus, poxvirus, vaccinia, poliovirus, Salmonella or BCG and thereafter administering the vector.

As stated above, the afore-mentioned SSSP's are designed for incorporation into vaccines wherein the synthetic peptides may be arranged in a variety of optimized combinations involving tandems, mirror images and repeating sequences. This strategy, well known to those skilled in the art, broadens the size of the antigen and extends a certain degree of flexibility for the immune systems of various individuals to respond while maintaining the focus on the selected, 3 dimensionally conserved residues. Since the residues I, M and L decrease immunogenicity, it is preferred that they be omitted in orderto augment and further optimize the immune responses. Although tryptophan also has poor antigenicity, it is preferred to retain this residue in the selected SSSP's because of the critical role of Trp427 which is required for CD4 binding.

The surface simulation synthetic peptides of the present invention may be chemically synthesized in accordance with any of the currently accepted protein synthesis methods well known to the art. For example, an automated ABI 430A solid-phase peptide synthesizer may by utilized. In addition, the SSSP sequences can be translated to nucleic acid sequences for mass production based upon recombinant DNA technology.

The SSSP's provided by the method of the present invention are especially useful in the production of vaccines for both prophylactic and therapeutic treatments. In other words, the vaccines incorporating the SSSP's of the present invention will not only have effect in preventing HIV infection in HIV negative individuals, but also provide an effective treatment in HIV-1 infected persons. As mentioned above, the SSSP's of the present invention define amino acid targets useful for the vaccines as well as diagnostic testing. Those skilled in the art are well aware of methods for utilizing amino acid residue sequences, such as those provided in the present SSSP's in order to produce vaccines. Effective vaccine design necessitates the usual and customary testing for the determination of optimal vectors and/or adjuvants for use of the particular vaccine, incorporating said SSSP targets, for administration to human subjects. The vaccines provided hereby may also further contain auxiliary substances such as, for example, wetting or emulsifying agents, pH buffering agents, or adjuvants such as, for example, Freund's adjuvant or aluminum phosphate to enhance antigenicity.

It is further contemplated that, for HIV-1 infected individuals, a vaccine utilizing the SSSP's of the present invention can be further optimized by co-administering a protease inhibitor containing anti-retroviral regimen for at least fourteen days. The reasons for this strategy is based upon the fact that the effectiveness of an immune response depends largely on antigen presentation. Whether type I or type 11 immune response in considered, antigen presentation is a key step in the process. Antigen presentation is an elaborate process that involves many different proteins in various compartments of the cell. The HIV protease enzyme is known to also digest a number of non-viral substrates including actin, Alzheimer amyloid precursor protein, clamodulin, insulin B chain, pro-IL-1-beta and troponin C Whether or not the HIV protease enzyme actually digests any molecule involved in antigen presentation is not known. It is known however, that immune cells from patients who are treated with a protease inhibitor are better at antigen presentation than cells from patients who did not include PI in their regimen. (ref. 13) Moreover computer analyses of molecules involved in antigen presentation reveal that these immune molecules have many potential sites for digestion by the HIV-1 protease enzyme. Therefore, where HIV-infected individuals are concerned, the use of a protease inhibitor will likely improve the immune response to the vaccine by optimizing antigen presentation.

As stated above, the present invention also contemplates utilization of the SSSP's provided thereby for diagnostic reagents and kits useful for detecting antibodies specific to hyper-variable viral pathogens such as HIV-1. Such kits may be comprised, for example, of a surface; at least one of the SSSP's provided hereby immobilized on said surface; a means for bringing into contact the antibodies to be detected and said SSSP(s) so as to allow formation of a complex; and a means for detecting the complex formed thereby. In regards to detecting antibodies specific to HIV-1, the present invention contemplates a kit comprising a surface; at least one SSSP specific for at least one non-sequential epitope of gp120 immobilized on said surface; a means for contacting said at least one SSSP and said antibody to be detected so as to allow formation of a complex; and a means for detecting the complex formed thereby. Such diagnostic kits enable determination as to whether an individual has acquired HIV-1 antibodies as a result of HIV infection or adequate vaccination In addition, such kits provide quantitative analyses to allow determination as to whether a vaccinated person has responded with adequate amount of protective antibodies against HIV-1.

### REFERENCES

1. Profy, A.T., P.S. Salinas, L.I. Eckler, N.M. Dunlop, P.L. Nara, and S.D. Putney, 1990. Epitopes recognized by the neutralizing antibodies of an HIV-1 infected individual. J. Immunol. 144:4641)
2. Moore JP, McCutchan FE, Poon SW, Mascola J, Liu J, Cao Y, Ho DD. (1994). Exploration of antigenic variation in gp120 from clades A through F of human immunodeficiency virus type 1 by using monoclonal antibodies. J Virol 1994 Dec;68(12):8350-64
3. VanCott TC, Bethke FR, Burke DS, Redfield RR, Birx DL: Lack of induction of antibodies specific for conserved, discontinuous epitopes of HIV-1 envelope glycoproteins by candidate aids vaccines. J Immunol 1995: 155:4100-4110
4. Olshevsky, V., Helseth, M., Furman, C., Li, J., Haseltine, W., and Sodroski, J. (1990). Identification of individual human immunodeficiency virus type 1 gp amino acids important for CD4 receptor binding. J. Virology. 64,12:5101-5707
5. Reed, J. Kinzel, B. 1994. Inhibitor of the conformational switch involved in CD4 binding by the end. glycoprotein gp120 from HIV I. Biochemistry 30: 4521-4528.
6. Kwong, P.D., Wyatt, R., Robinson, J. Sweet, R.W., Sdroski, J. Hendrickson, W.W. 1998. Structure of an HIV gp120 envelope glycoprotein in complex with the CD4 receptor and neutralizing human antibody. Nature. 393:648-655
7. Willey, R.L., Smith, D.H., Lasky, L.A., Theodore, S.T., Earl, P.L., Moss, B., Kapon, D.J., Martin, M.A. 1988. In vitro mutagenesis identifies a region within the envelope gene of the human immunodeficiency virus that is critical for infectivity. J. Virology. 62, 1:139-147.
8. Tschashler E., Buchow H., Gallo RC, Reitz MS Jr. 1990 Functional contribution of cysteine residues to the human immunodeficiency virus type 1 envelope. J. Virol. 1990 May;64(5):2250-9.
9. Chirmule N, Kalyanaraman V.S., Slade H, Oyaizu N, Pahwa S. 1990. Requirement of the T cell receptor for antigen presentation by T lymphocytes. Effect of envelope glycoproteins of HIV-1 on antigen presentation by T cells. Clin. Exp. Immuno. 1990 May;80(2):161-6.
10. Kang Y, Melo EF, Scott DW. 1998. An ongoing immune response to HIV envelope gp120 in human CD4-transgenic mice contributes to T cell decline upon intravenous administration of gp120. Eur J Immonol 1998 Aug;28(8):2253-64.
11. Waring, P, Beaver, J 1996 Gliotoxin and related epipolythiodioxopiperazines. Gen Pharmacol 1996 Dec;27(8):1311-6
12. Thali, M. Furman, C., Ho, D.D., Robinson, J. Tilley, S., Pinter, A., Sodroski, J. 1992. Discontinuous, conserved neutralization epitopes overlapping the CD4-binding region of human immunodeficiency virus type 1 gp120 envelope glycoprotein. J. Virology. 66,9:5635-5641.
13. Kelleher AD, Carr A, Zaunders J, Cooper, DA. 1996. Alterations in the immune response of human immunodeficiency virus (HIV)-infected subjects treated with HIV-specific protease inhibitor ritonavir. J Infect Dis. 1996 Feb;173(2):321-9.
14. Atassi, MZ, Bixlerr GS Jr., Yokoi T. 1989 Conformation-dependent recognition of a protein by T cells requires presentation without processing. Biochem J. 1989 May 1;259(3):731-5
15. Atassi, MZ, Manshouri T. 1993 Design of peptide enzymes (pepzymes): suface-simulation synthetic peptides that mimic the chymotrypsin and trypsin active sites exhibit the activity and specificity of the respective enzyme. Proc Natl Acad Sci U.S.A. 1993 Sep 1;90(17):8282-6.

## Claims

1. A method for configuring and preparing surface simulation synthetic peptides effective in the diagnosis and treatment of HIV-1, a hyper-variable viral pathogen, said method comprising:
selecting gp120, a protein of said subject pathogenic HIV-1 virus required for viral pathogenicity having an amino acid sequence;
identifying, within the amino acid sequence of said protein, non-sequential conserved residues required for viral pathogenicity;
complexing the protein with a natural binding site located on a CD4+ T lymphocyte;
subjecting said complexed protein to an imaging scan so as to obtain and record 3 dimensional and spatial relationship data in regards to each of said conserved residues;
subjecting said 3 dimensional image data obtained by said imaging scan to computer analysis via protein analysis software in order to obtain a functional map thereof;
determining, by means of said analytic software program, a 3 dimensional position for each of said non-sequential conserved residues within said viral protein and spatial relationships thereamong;
designing sequences for surface simulation synthetic peptides having amino acids ordered therein so that each sequential amino acid incorporated within the synthetic peptide corresponds to a native conserved amino acid within an epitope which is spatially positioned, within the complexed gp120 protein, no more than approximately 10 Angstroms from its most proximate, conserved native residue, and thus the residues incorporated within the surface simulation synthetic peptides correspond to and approximate the identified 3-dimensional relationships of selected conserved, non-sequential residues
in regards to relative 3 dimensional proximity;
preparing surface simulation synthetic peptides whose amino acid sequences are ordered so that each sequential amino acid incorporated within the synthetic peptide corresponds to and is representative of a native conserved amino acid which is spatially positioned, in 3-D, within the complexed gp120 protein, no more than 10 Angstroms from its most proximate adjacent conserved native residue and thus corresponds to the identified 3-dimensional spatial relationships of said conserved residues required for viral pathogenicity.

2. The method of claim 1 wherein the selected viral protein is necessary for viral infectivity.

3. The method of claim 1 wherein the selected viral protein component is necessary for viral binding.

4. The method of claim 1 wherein said imaging scan is obtained by means of x-ray crystallography.

5. The method of claim 1 wherein the identified non-sequential conserved residues are required in order to provide viral infectivity.

6. The method of claim 1 wherein the identified conserved residues are necessary in order to provide viral binding.

7. The method of claim 1 wherein the identified non-sequential conserved residues provide said viral protein with a 3 dimensional viral protein configuration required for viral pathogenicity.

8. The method of claim 1 wherein surface simulation synthetic peptides are arranged in tandems, mirror images and tandem repeats.

9. The method of claim 1 further comprising utilizing a computer search engine in order to compare said designed surface simulation synthetic peptides with human gene, protein and peptide databases in order to identify and eliminate any such synthetic peptide having an amino acid sequence homologous to peptides in such databases.

10. The method of claim 1 further comprising analyzing and evaluating said surface simulation synthetic peptides for antigenicity, hydropathy, accessibility and flexibility thereof utilizing protein analysis software and thereafter modifying the sequence of said surface simulation synthetic peptide in accordance with the results of said software analysis in order to optimize the antigenicity and accessibility thereof.

11. The method of claim 9 further comprising analyzing and evaluating said surface simulation synthetic peptides for antigenicity, hydropathy, accessibility and flexibility thereof utilizing protein analysis software and thereafter modifying the sequence of said surface simulation synthetic peptide in accordance with the results of said software analysis in order to optimize the antigenicity and accessibility thereof.

12. The method of claim 1 wherein said non-sequential conserved residues are selected from the groups consisting of:
Thr257, Asp368, Glu370, Trp427, Asp457;
Arg419, Lys421, Gln422;
Asn262;
Met434; and
Cys385, Cys418, Cys331, Cys296, Cys418.

13. The method of claim 12 wherein said surface simulation synthetic peptides are designed so as to incorporate tandems, mirror images and tandem repeats of said non-sequential conserved amino acid sequences.

14. A method for configuring and preparing surface simulation synthetic peptides effective in the diagnosis and treatment of H1V-1, a hyper-variable viral pathogen, said method comprising:
selecting gp120, a protein of said subject pathogenic HIV-1 virus required for viral pathogenicity having an amino acid sequence;
identifying, within the amino acid sequence of said protein, non-sequential conserved residues required for viral pathogenicity;
complexing the protein with a natural binding site located on a CD4+ T lymphocyte;
subjecting said complexed protein to an imaging scan so as to obtain and record 3 dimensional and spatial relationship data in regards to each of said conserved residues,
subjecting said 3 dimensional image data obtained by said imaging scan to computer analysis via protein analysis software in order to obtain a functional map thereof;
determining, by means of said analytic software program, a 3 dimensional position for each of said non-sequential conserved residues within said viral protein and spatial relationships thereamong;
designing sequences for surface simulation synthetic peptides having amino acids ordered therein so that each sequential amino acid incorporated within the synthetic peptide corresponds to a native conserved amino acid within an epitope which is spatially positioned, within the complexed gp120 protein, no more than approximately 10 Angstroms from its most proximate, conserved native residue, and thus the residues incorporated within the surface simulation synthetic peptides correspond to and approximate the identified 3-dimensional relationships of selected conserved, non-sequential residues in regards to relative 3 dimensional proximity;
utilizing a computer search engine in order to compare said designed surface simulation synthetic peptides with human gene, protein and peptide databases in order to identify and eliminate any such synthetic peptide having an amino acid sequence homologous to peptides in such databases;
further analyzing and evaluating said surface simulation synthetic peptides for antigenicity, hydropathy, accessibility and flexibility thereof utilizing protein analysis software;
modifying the sequence of said surface simulation synthetic peptide in accordance with the results of said software analysis in order to optimize the antigenicity and accessibility thereof;
preparing surface simulation synthetic peptides whose amino acid sequences are ordered so that each sequential amino acid incorporated within the synthetic peptide corresponds to and is representative of a native conserved amino acid which is spatially positioned, in respect to its 3-dimensional position within the complexed gp120 protein, no more than approximately 10 Angstroms from its most proximate, adjacent conserved residue and thus corresponds to the identified 3 dimensional spatial relationships of said conserved residues required for viral pathogenicity and whose amino acid sequences are further refined to provide optimized antigenicity.

15. The method of claim 14 wherein the selected viral protein is necessary for viral infectivity.

16. The method of claim 14 wherein the selected viral protein is necessary for viral binding.

17. The method of claim 14 wherein said imaging scan is obtained by means of x-ray crystallography.

18. The method of claim 14 wherein the identified non-sequential conserved residues are required in order to provide viral infectivity.

19. The method of claim 14 wherein the identified conserved residues are necessary in order to provide viral binding.

20. The method of claim 14 wherein the identified non-sequential conserved residues provide said viral protein with a 3 dimensional viral protein configuration required for viral pathogenicity.

21. The method of claim 14 wherein said surface synthetic simulation peptides are arranged in tandems, mirror images and tandem repeats.

22. The method of claim 14 wherein said non-sequential conserved residues are selected from the groups consisting of:
Thr257, Asp368, Glu370, Trp427, Asp457;
Arg419, Lys421, Gln422;
Asn262;
Met434; and
Cys385, Cys418, Cys331, Cys296, Cys418.

23. The method of claim 22 wherein said surface simulation synthetic peptides are designed so as to incorporate tandems, mirror images and tandem repeats of said non-sequential conserved amino acid sequences.

24. A surface simulation synthetic peptide having at least one amino acid sequence corresponding to a non-sequential conserved residue epitope of HIV-1 glycoprotein gp120 required for viral pathogenicity, said peptide comprised of a sequence selected from:
DWETCNNCRKEDW (SEQ ID NO:1)
NCRKEDWDEKRCN (SEQ ID NO:2)
DWETCNCTEWD (SEQ ID: NO: 3)
WDETCNCTEDWDETCN (SEQ ID: NO: 4)
NCRTEKQWQKETRCN (SEQ ID: NO: 5)
QWKERTCN (SEQ ID: NO: 6)
QKWETCCRKW (SEQ ID:NO: 7)
QKWDETCCRKW (SEQ ID: NO: 8)
DWETCN (SEQ ID: NO: 9)
DWETCCRKQWK (SEQ ID: NO: 10)
QKRCCTEDW (SEQ ID: NO: 11)
QKRCCNTEDWIIMQ (SEQ ID: NO: 12)
QKRCCTED (SEQ ID: NO: 13)
DWEKRCCNTEDWK (SEQ ID: NO: 14)
QKRCCTED (SEQ ID: NO: 15)
WQKRCCTEDW (SEQ ID: NO: 16)
KWETCCRK (SEQ ID: NO: 17)
KWETCCRKQ (SEQ ID: NO: 18)
TNCCRKET (SEQ ID: NO: 19)
DWETNCCRKQWK (SEQ ID: NO: 20)
IWDETNCCRKQIWK (SEQ ID: NO: 21)
IWDETNCCRKQNIWK (SEQ ID: NO: 22)
LPCRKQMIW (SEQ ID: NO: 23)
LPCRKQMIWDWETNC (SEQ ID: NO: 24)
LPCRIKQMIW (SEQ ID: NO: 25)
LPCRIKQMIWDWETNC (SEQ ID: NO: 26)
LPCRKQWDWETCNC (SEQ ID: NO: 27)
LPCCTEWD (SEQ ID: NO: 28)
LPCCTEWKR (SEQ ID: NO: 29)
LPCCCCRK (SEQ ID: NO: 30)
CCCCRKWETCCCCN (SEQ ID: NO: 31)
CCCCRKQWKETCCCCN (SEQ ID: NO: 32)
CCCCTEDWKQRCCCCPN (SEQ ID: NO: 33).

## Patentansprüche

1. Verfahren zum Konfigurieren und Herstellen von synthetischen Oberflächensimulationspeptiden, die bei der Diagnose und Behandlung von HIV-1, einem hypervariablen viralen Pathogen, wirksam sind, wobei das Verfahren folgendes umfasst:
man wählt gp120 aus, ein Protein dieses pathogenen HIV-1-Virus, das für die virale Pathogenizität erforderlich ist und eine bestimmte Aminosäuresequenz aufweist;
man identifiziert innerhalb der Aminosäuresequenz dieses Proteins nichtsequenzielle, konservierte Reste, die für die virale Pathogenizität erforderlich sind;
man komplexiert das Protein mit einer natürlichen Bindungsstelle, die sich an einem CD4⁺-T-Lymphozyten befindet;
man unterwirft das komplexierte Protein einem bildgebenden Scanvorgang, um Daten über die dreidimensionalen und räumlichen Beziehungen bezüglich der einzelnen konservierten Reste zu gewinnen und aufzuzeichnen;
man unterwirft die durch den bildgebenden Scanvorgang erhaltenen dreidimensionalen Abbildungsdaten über eine Proteinanalysen-Software einer Computeranalyse, um daraus eine funktionelle Karte zu erhalten;
man bestimmt mittels des analytischen Softwareprogramms eine dreidimensionale Position für jeden der nicht-sequenziellen, konservierten Reste innerhalb des viralen Proteins und die zwischen diesen bestehenden räumlichen Beziehungen;
man konstruiert Sequenzen für synthetische Oberflächensimulationspeptide mit darin so geordneten Aminosäuren, dass jede in das synthetische Peptid eingebaute sequenzielle Aminosäure einer nativen, konservierten Aminosäure innerhalb eines Epitops entspricht, die räumlich innerhalb des komplexierten gp120-Proteins in einer Entfernung von nicht mehr als etwa 10 Ǻngström von ihrem nächstliegenden, konservierten, nativen Rest positioniert ist, und dass somit die in die synthetischen Oberflächensimulationspeptide eingebauten Reste den identifizierten dreidimensionalen Beziehungen von ausgewählten konservierten, nicht-sequenziellen Resten in Bezug auf die relative dreidimensionale Nähe entsprechen und diesen angenähert sind;
man stellt synthetische Oberflächensimulationspeptide her, deren Aminosäuresequenzen so geordnet sind, dass jede in das synthetische Peptid eingebaute sequenzielle Aminosäure einer nativen, konservierten Aminosäure entspricht und für diese repräsentativ ist, die räumlich in dreidimensionaler Anordnung im komplexierten gp120-Protein in einer Entfernung von nicht mehr als 10 Ǻngström von ihrem nächstliegenden, benachbarten, konservierten, nativen Rest positioniert ist und somit den identifizierten dreidimensionalen räumlichen Beziehungen der für die virale Pathogenizität erfoderlichen konservierten Reste entspricht.

2. Verfahren nach Anspruch 1, wobei das ausgewählte virale Protein für die virale Infektiosität erforderlich ist.

3. Verfahren nach Anspruch 1, wobei die ausgewählte virale Proteinkomponente für die virale Bindung erforderlich ist.

4. Verfahren nach Anspruch 1, wobei der bildgebende Scanvorgang durch Röntgenkristallographie erfolgt.

5. Verfahren nach Anspruch 1, wobei die identifizierten nicht-sequenziellen, konservierten Reste zur Bereitstellung der viralen Infektiosität erforderlich sind.

6. Verfahren nach Anspruch 1, wobei die identifizierten konservierten Reste zur Bereitstellung der viralen Bindung erforderlich sind.

7. Verfahren nach Anspruch 1, wobei die identifizierten nicht-sequenziellen, konservierten Reste dem viralen Protein eine dreidimensionale virale Proteinkonfiguration verleihen, die für die virale Pathogenizität erforderlich ist.

8. Verfahren nach Anspruch 1, wobei die synthetischen Oberflächensimulationspeptide als Tandems, Spiegelbilder und Tandem-Wiederholungen angeordnet sind.

9. Verfahren nach Anspruch 1, ferner umfassend die Verwendung einer Computer-Suchmaschine, um die konstruierten synthetischen Oberflächensimulationspeptide mit humanen Gen-, Protein- und Peptid-Datenbanken zu vergleichen, um etwaige synthetische Peptide, die eine zu Peptiden in diesen Datenbanken homologe Aminosäuresequenz aufweisen, zu identifizieren und zu entfernen.

10. Verfahren nach Anspruch 1, ferner umfassend die Analyse und die Bewertung der synthetischen Oberflächensimulationspeptide in Bezug auf Antigenizität, Hydropathie, Zugänglichkeit und Flexibilität unter Verwendung einer Proteinanalysen-Software und anschließend das Modifizieren der Sequenz des synthetischen Oberflächensimulationspeptids gemäß den Ergebnissen der Software-Analyse, um dessen Antigenizität und Zugänglichkeit zu optimieren.

11. Verfahren nach Anspruch 9, ferner umfassend die Analyse und die Bewertung der synthetischen Oberflächensimulationspeptide in Bezug auf Antigenizität, Hydropathie, Zugänglichkeit und Flexibilität unter Verwendung einer Proteinanalysen-Software und anschließend das Modifizieren der Sequenz des synthetischen Oberflächensimulationspeptids gemäß den Ergebnissen der Software-Analyse, um dessen Antigenizität und Zugänglichkeit zu optimieren.

12. Verfahren nach Anspruch 1, wobei die nicht-sequenziellen konservierten Reste aus den Gruppen ausgewählt werden, die bestehen aus:
Thr257, Asp368, Glu370, Trp427, Asp457;
Arg419, Lys421, Gln422;
An262;
Met434; und
Cys385, Cys418, Cys331, Cys296 und Cys 418.

13. Verfahren nach Anspruch 12, wobei die synthetischen Oberflächensimulationspeptide so konstruiert sind, dass sie Tandems, Spiegelbilder und Tandem-Wiederholungen der nicht-sequenziellen, konservierten Aminosäuresequenzen enthalten.

14. Verfahren zum Konfigurieren und Herstellen von synthetischen Oberflächensimulationspeptiden, die bei der Diagnose und Behandlung von HIV-1, einem hypervariablen viralen Pathogen, wirksam sind, wobei das Verfahren folgendes umfasst:
man wählt gp120 aus, ein Protein dieses pathogenen HIV-1-Virus, das für die virale Pathogenizität erforderlich ist und eine bestimmte Aminosäuresequenz aufweist;
man identifiziert innerhalb der Aminosäuresequenz dieses Proteins nichtsequenzielle, konservierte Reste, die für die virale Pathogenizität erforderlich sind;
man komplexiert das Protein mit einer natürlichen Bindungsstelle, die sich an einem CD4⁺-T-Lymphozyten befindet;
man unterwirft das komplexierte Protein einem bildgebenden Scanvorgang, um Daten über die dreidimensionalen und räumlichen Beziehungen bezüglich der einzelnen konservierten Reste zu gewinnen und aufzuzeichnen;
man unterwirft die durch den bildgebenden Scanvorgang erhaltenen dreidimensionalen Abbildungsdaten über eine Proteinanalysen-Software einer Computeranalyse, um daraus eine funktionelle Karte zu erhalten;
man bestimmt mittels des analytischen Softwareprogramms eine dreidimensionale Position für jeden der nicht-sequenziellen, konservierten Reste innerhalb des viralen Proteins und die zwischen diesen bestehenden räumlichen Beziehungen;
man konstruiert Sequenzen für synthetische Oberflächensimulationspeptide mit darin so geordneten Aminosäuren, dass jede in das synthetische Peptid eingebaute sequenzielle Aminosäure einer nativen konservierten Aminosäure innerhalb eines Epitops entspricht, die räumlich innerhalb des komplexierten gp120-Proteins in einer Entfernung von nicht mehr als etwa 10 Ǻngström von ihrem nächstliegenden, konservierten, nativen Rest positioniert ist, und dass somit die in die synthetischen Oberflächensimulationspeptide eingebauten Reste den identifizierten dreidimensionalen Beziehungen von ausgewählten konservierten, nicht-sequenziellen Resten in Bezug auf die relative dreidimensionale Nähe entsprechen und diesen angenähert sind;
man bedient sich ferner einer Computer-Suchmaschine, um die konstruierten synthetischen Oberflächensimulationspeptide mit humanen Gen-, Protein- und Peptid-Datenbanken zu vergleichen, um etwaige synthetische Peptide, die eine zu Peptiden in diesen Datenbanken homologe Aminosäuresequenz aufweisen, zu identifizieren und zu entfernen.
man führt ferner eine Analyse und Bewertung der synthetischen Oberflächensimulationspeptide in Bezug auf Antigenizität, Hydropathie, Zugänglichkeit und Flexibilität durch, wobei man die Proteinanalysen-Software verwendet;
man modifiziert die Sequenz des synthetischen Oberflächensimulationspeptids gemäß den Ergebnissen der Software-Analyse, um dessen Antigenizität und Zugänglichkeit zu optimieren;
man stellt synthetische Oberflächensimulationspeptide her, deren Aminosäuresequenzen so geordnet sind, dass jede in das synthetische Peptid eingebaute sequenzielle Aminosäure einer nativen konservierten Aminosäure entspricht und für diese repräsentativ ist, die räumlich in dreidimensionaler Anordnung im komplexierten gp120-Protein in einer Entfernung von nicht mehr als etwa 10 Ǻngström von ihrem nächstliegenden, benachbarten, konservierten, nativen Rest positioniert ist und somit den identifizierten dreidimensionalen räumlichen Beziehungen der für die virale Pathogenizität erfoderlichen konservierten Reste entspricht.

15. Verfahren nach Anspruch 14, wobei das ausgewählte virale Protein für die virale Infektiosität erforderlich ist.

16. Verfahren nach Anspruch 14, wobei das ausgewählte virale Protein für die virale Bindung erforderlich ist.

17. Verfahren nach Anspruch 14, wobei der bildgebende Scanvorgang durch Röntgenkristallographie erfolgt.

18. Verfahren nach Anspruch 14, wobei die identifizierten nicht-sequenziellen, konservierten Reste zur Bereitstellung der viralen Infektiosität erforderlich sind.

19. Verfahren nach Anspruch 14, wobei die identifizierten konservierten Reste zur Bereitstellung der viralen Bindung erforderlich sind.

20. Verfahren nach Anspruch 14, wobei die identifizierten nicht-sequenziellen, konservierten Reste dem viralen Protein eine dreidimensionale virale Proteinkonfiguration verleihen, die für die virale Pathogenizität erforderlich ist.

21. Verfahren nach Anspruch 14, wobei die synthetischen Oberflächensimulationspeptide als Tandems, Spiegelbilder und Tandem-Wiederholungen angeordnet sind.

22. Verfahren nach Anspruch 14, wobei die nicht-sequenziellen konservierten Reste aus den Gruppen ausgewählt werden, die bestehen aus:
Thr257, Asp368, Glu370, Trp427, Asp457;
Arg419, Lys421, Gln422;
Asn262;
Met434; und
Cys385, Cys418, Cys331, Cys296 und Cys 418.

23. Verfahren nach Anspruch 22, wobei die synthetischen Oberflächensimulationspeptide so konstruiert sind, dass sie Tandems, Spiegelbilder und Tandem-Wiederholungen der nicht-sequenziellen, konservierten Aminosäuresequenzen enthalten.

24. Synthetisches Oberflächensimulationspeptid mit mindestens einer Aminosäuresequenz, die einem nicht-sequenziellen, konservierten Reste-Epitop von HIV-1-Glycoprotein gp120, das für die virale Pathogenizität erforderlich ist, entspricht, wobei das Peptid eine Sequenz umfasst, die ausgewählt ist aus:
DWETCNNCRKEDW (SEQ ID: NO: 1)
NCRKEDWDEKRCN (SEQ ID: NO: 2)
DWETCNCTEWD (SEQ ID: NO: 3)
WDETCNCTEDWDETCN (SEQ ID: NO: 4)
NCRTEKQWQKETRCN (SEQ ID: NO: 5)
QWKERTCN (SEQ ID: NO: 6)
QKWETCCRKW (SEQ ID: NO: 7)
QKWDETCCRKW (SEQ ID: NO: 8)
DWETCN (SEQ ID: NO: 9)
DWETCCRKQWK (SEQ ID: NO: 10)
QKRCCTEDW (SEQ ID: NO: 11)
QKRCCNTEDWIIMQ (SEQ ID: NO: 12)
QKRCCTED (SEQ ID: NO: 13)
DWEKRCCNTEDWK (SEQ ID: NO: 14)
QKRCCTED (SEQ ID: NO: 15)
WQKRCCTEDW (SEQ ID: NO: 16)
KWETCCRK (SEQ ID: NO: 17)
KWETCCRKQ (SEQ ID: NO: 18)
TNCCRKET (SEQ ID: NO: 19)
DWETNCCRKQWK (SEQ ID: NO: 20)
IWDETNCCRKQIWK (SEQ ID: NO: 21)
IWDETNCCRKQNIWK (SEQ ID: NO: 22)
LPCRKQMIW (SEQ ID: NO: 23)
LPCRKQMIWDWETNC (SEQ ID: NO: 24)
LPCRIKQMIW (SEQ ID: NO: 25)
LPCRIKQMIWDWETNC (SEQ ID: NO: 26)
LPCRKQWDWETCNC (SEQ ID: NO: 27)
LPCCTEWD (SEQ ID: NO: 28)
LPCCTEWKR (SEQ ID: NO: 29)
LPCCCCRK (SEQ ID: NO: 30)
CCCCRKWETCCCCN (SEQ ID: NO: 31)
CCCCRKQWKETCCCCN (SEQ ID: NO: 32)
CCCCTEDWKQRCCCCPN (SEQ ID: NO: 33)

## Revendications

1. Procédé permettant de configurer et de préparer des peptides synthétiques de simulation de surface, efficaces dans le diagnostic et le traitement du virus pathogène hypervariable qu'est le virus VIH-1, lequel procédé comporte les étapes suivantes :
- sélectionner la protéine gp120, une protéine dudit virus pathogène VIH-1 qui est nécessaire pour la pathogénicité du virus et qui présente une séquence d'acides aminés ;
- identifier, au sein de la séquence d'acides aminés de cette protéine, des résidus conservés non-successifs nécessaires pour la pathogénicité du virus ;
- complexer la protéine avec un site de liaison naturel situé sur un lymphocyte T CD4+ ;
- prendre une image de ladite protéine complexée, de façon à obtenir et enregistrer des données de relations spatiales dans les trois dimensions concernant chacun desdits résidus conservés ;
- soumettre les données d'image en trois dimensions obtenues lors de ladite prise d'image à une analyse par ordinateur effectuée à l'aide d'un logiciel d'analyse de protéines, de manière à en obtenir une carte fonctionnelle ;
- déterminer, à l'aide d'un programme dudit logiciel d'analyse, la position dans les trois dimensions de chacun desdits résidus conservés non-successifs au sein de ladite protéine virale, ainsi que leurs relations spatiales les uns aux autres ;
- concevoir des séquences de peptides synthétiques de simulation de surface comportant des acides aminés qui y sont ordonnés de telle sorte que chaque acide aminé successif incorporé dans le peptide synthétique corresponde à un acide aminé natif conservé au sein d'un épitope qui se situe dans l'espace, au sein de la protéine gp120 complexée, à au plus environ 10 angströms du résidu natif conservé le plus proche et qu'ainsi, les résidus incorporés dans les peptides synthétiques de simulation de surface correspondent aux résidus conservés non-successifs choisis et, pour ce qui est de leur proximité relative dans les trois dimensions, aient à peu près les relations tridimensionnelles identifiées pour ceux-ci ;
- et préparer des peptides synthétiques de simulation de surface dont les séquences d'acides aminés sont ordonnées de telle sorte que chaque acide aminé successif incorporé dans le peptide synthétique corresponde à un acide aminé natif conservé et soit représentatif de cet acide aminé qui se situe dans l'espace en trois dimensions, au sein de la protéine gp120 complexée, à au plus environ 10 angströms du résidu natif conservé adjacent le plus proche et qu'ainsi, ses relations spatiales correspondent aux relations spatiales tridimensionnelles identifiées pour lesdits résidus conservés nécessaires pour la pathogénicité du virus.

2. Procédé conforme à la revendication 1, dans lequel la protéine virale sélectionnée est nécessaire pour l'infectiosité du virus.

3. Procédé conforme à la revendication 1, dans lequel la protéine virale sélectionnée est nécessaire pour la liaison du virus.

4. Procédé conforme à la revendication 1, dans lequel ladite image est obtenue par cristallographie aux rayons X.

5. Procédé conforme à la revendication 1, dans lequel les résidus conservés non-successifs identifiés sont nécessaires pour l'infectiosité du virus.

6. Procédé conforme à la revendication 1, dans lequel les résidus conservés identifiés sont nécessaires pour la liaison du virus.

7. Procédé conforme à la revendication 1, dans lequel les résidus conservés non-successifs identifiés confèrent à ladite protéine virale une configuration tridimensionnelle de protéine virale nécessaire pour la pathogénicité du virus.

8. Procédé conforme à la revendication 1, dans lequel les peptides synthétiques de simulation de surface sont disposés en tandem, en images dans un miroir ou en répétitions en tandem.

9. Procédé conforme à la revendication 1, qui comporte en outre le fait d'utiliser un moteur de recherche informatique afin de comparer lesdits peptides synthétiques de simulation de surface ainsi conçus avec les banques de données de gènes, protéines et peptides humains pour identifier et éliminer tout peptide synthétique qui aurait une séquence d'acide aminé homologue à celle de l'un des peptides présents dans ces banques de données.

10. Procédé conforme à la revendication 1, qui comporte en outre le fait d'analyser lesdits peptides synthétiques de simulation de surface pour en évaluer l'antigénicité, l'hydropathie, l'accessibilité et la flexibilité, en utilisant un logiciel d'analyse de protéines, et le fait de modifier ensuite la séquence dudit peptide synthétique de simulation de surface, en fonction des résultats fournis par cette analyse par logiciel, afin d'optimiser l'antigénicité et l'accessibilité dudit peptide.

11. Procédé conforme à la revendication 9, qui comporte en outre le fait d'analyser lesdits peptides synthétiques de simulation de surface pour en évaluer l'antigénicité, l'hydropathie, l'accessibilité et la flexibilité, en utilisant un logiciel d'analyse de protéines, et le fait de modifier ensuite la séquence dudit peptide synthétique de simulation de surface, en fonction des résultats fournis par cette analyse par logiciel, afin d'optimiser l'antigénicité et l'accessibilité dudit peptide.

12. Procédé conforme à la revendication 1, dans lequel lesdits résidus conservés non-successifs sont choisis parmi les ensembles constitués par les suivants :
- Thr257, Asp368, Glu370, Trp427, Asp457 ;
- Arg419, Lys421, Gln422 ;
- Asn262 ;
- Met434 ;
- et Cys385, Cys418, Cys331, Cys296, Cys418.

13. Procédé conforme à la revendication 12, dans lequel lesdits peptides synthétiques de simulation de surface sont conçus de manière à incorporer des tandems, des images dans un miroir ou des répétitions en tandem desdites séquences d'acides aminés conservés non-successifs.

14. Procédé permettant de configurer et de préparer des peptides synthétiques de simulation de surface, efficaces dans le diagnostic et le traitement du virus pathogène hypervariable qu'est le virus VIH-1, lequel procédé comporte les étapes suivantes :
- sélectionner la protéine gp120, une protéine dudit virus pathogène VIH-1 qui est nécessaire pour la pathogénicité du virus et qui présente une séquence d'acides aminés ;
- identifier, au sein de la séquence d'acides aminés de cette protéine, des résidus conservés non-successifs nécessaires pour la pathogénicité du virus ;
- complexer la protéine avec un site de liaison naturel situé sur un lymphocyte T CD4+ ;
- prendre une image de ladite protéine complexée, de façon à obtenir et enregistrer des données de relations spatiales dans les trois dimensions concernant chacun desdits résidus conservés ;
- soumettre les données d'image en trois dimensions obtenues lors de ladite prise d'image à une analyse par ordinateur effectuée à l'aide d'un logiciel d'analyse de protéines, de manière à en obtenir une carte fonctionnelle ;
- déterminer, à l'aide d'un programme dudit logiciel d'analyse, la position dans les trois dimensions de chacun desdits résidus conservés non-successifs au sein de ladite protéine virale, ainsi que leurs relations spatiales les uns aux autres ;
- concevoir des séquences de peptides synthétiques de simulation de surface comportant des acides aminés qui y sont ordonnés de telle sorte que chaque acide aminé successif incorporé dans le peptide synthétique corresponde à un acide aminé natif conservé au sein d'un épitope qui se situe dans l'espace, au sein de la protéine gp120 complexée, à au plus environ 10 angströms du résidu natif conservé le plus proche et qu'ainsi, les résidus incorporés dans les peptides synthétiques de simulation de surface correspondent aux résidus conservés non-successifs choisis et, pour ce qui est de leur proximité relative dans les trois dimensions, aient à peu près les relations tridimensionnelles identifiées pour ceux-ci ;
- utiliser un moteur de recherche informatique afin de comparer lesdits peptides synthétiques de simulation de surface ainsi conçus avec les banques de données de gènes, protéines et peptides humains pour identifier et éliminer tout peptide synthétique qui aurait une séquence d'acide aminé homologue à celle de l'un des peptides présents dans ces banques de données ;
- analyser ensuite lesdits peptides synthétiques de simulation de surface pour en évaluer l'antigénicité, l'hydropathie, l'accessibilité et la flexibilité, en utilisant un logiciel d'analyse de protéine ;
- modifier la séquence dudit peptide synthétique de simulation de surface, en fonction des résultats fournis par cette analyse par logiciel, afin d'optimiser l'antigénicité et l'accessibilité dudit peptide ;
- et préparer des peptides synthétiques de simulation de surface dont les séquences d'acides aminés sont ordonnées de telle sorte que chaque acide aminé successif incorporé dans le peptide synthétique corresponde à un acide aminé natif conservé et soit représentatif de cet acide aminé qui se situe dans l'espace en trois dimensions, au sein de la protéine gp120 complexée, à au plus environ 10 angströms du résidu natif conservé adjacent le plus proche et qu'ainsi, ses relations spatiales correspondent aux relations spatiales tridimensionnelles identifiées pour lesdits résidus conservés nécessaires pour la pathogénicité du virus, et dont les séquences d'acides aminés sont en outre affinées de manière à donner une antigénicité optimisée.

15. Procédé conforme à la revendication 14, dans lequel la protéine virale sélectionnée est nécessaire pour l'infectiosité du virus.

16. Procédé conforme à la revendication 14, dans lequel la protéine virale sélectionnée est nécessaire pour la liaison du virus.

17. Procédé conforme à la revendication 14, dans lequel ladite image est obtenue par cristallographie aux rayons X.

18. Procédé conforme à la revendication 14, dans lequel les résidus conservés non-successifs identifiés sont nécessaires pour l'infectiosité du virus.

19. Procédé conforme à la revendication 14, dans lequel les résidus conservés identifiés sont nécessaires pour la liaison du virus.

20. Procédé conforme à la revendication 14, dans lequel les résidus conservés non-successifs identifiés confèrent à ladite protéine virale une configuration tridimensionnelle de protéine virale nécessaire pour la pathogénicité du virus.

21. Procédé conforme à la revendication 14, dans lequel les peptides synthétiques de simulation de surface sont disposés en tandem, en images dans un miroir ou en répétitions en tandem.

22. Procédé conforme à la revendication 14, dans lequel lesdits résidus conservés non-successifs sont choisis parmi les ensembles constitués par les suivants :
- Thr257, Asp368, Glu370, Trp427, Asp457 ;
- Arg419, Lys421, Gln422 ;
- Asn262 ;
- Met434 ;
- et Cys385, Cys418, Cys331, Cys296, Cys418.

23. Procédé conforme à la revendication 22, dans lequel lesdits peptides synthétiques de simulation de surface sont conçus de manière à incorporer des tandems, des images dans un miroir ou des répétitions en tandem desdites séquences d'acides aminés conservés non-successifs.

24. Peptide synthétique de simulation de surface présentant au moins une séquence d'acide aminé qui correspond à un épitope à résidus conservés non-successifs de la glycoprotéine gp120 du virus VIH-1, nécessaire pour la pathogénicité du virus, lequle peptide est constitué d'une séquence choisie parmi les suivantes :
DWETCNNCRKEDW (Séquence N° 1)
NCRKEDWDEKRCN (Séquence N° 2)
DWETCNCTEWD (Séquence N° 3)
WDETCNCTEDWDETCN (Séquence N° 4)
NCRTEKQWQKETRCN (Séquence N° 5)
QWKERTCN (Séquence N° 6)
QKWETCCRKW (Séquence N° 7)
QKWDETCCRKW (Séquence N° 8)
DWETCN (Séquence N° 9)
DWETCCRKQWK (Séquence N° 10)
QKRCCTEDW (Séquence N° 11)
QKRCCNTEDWIIMQ (Séquence N° 12)
QKRCCTED (Séquence N° 13)
DWEKRCCNTEDWK (Séquence N° 14)
QKRCCTED (Séquence N° 15)
WQKRCCTEDW (Séquence N° 16)
KWETCCRK (Séquence N° 17)
KWETCCRKQ (Séquence N° 18)
TNCCRKET (Séquence N° 19)
DWETNCCRKQWK (Séquence N° 20)
IWDETNCCRKQIWK (Séquence N° 21)
IWDETNCCRKQNIWK (Séquence N° 22)
LPCRKQMIW (Séquence N° 23)
LPCRKQMIWDWETNC (Séquence N° 24)
LPCRIKQMIW (Séquence N° 25)
LPCRIKQMIWDWETNC (Séquence N° 26)
LPCRKQWDWETCNC (Séquence N° 27)
LPCCTEWD (Séquence N° 28)
LPCCTEWKR (Séquence N° 29)
LPCCCCRK (Séquence N° 30)
CCCCRKWETCCCCN (Séquence N° 31)
CCCCRKQWKETCCCCN (Séquence N° 32)
CCCCTEDWKQRCCCCPN (Séquence N° 33)
